(19) Europäisches Patentamt
European Patent Office
Office européen des brevets

(11) **EP 3 939 657 A1**

(12) **EUROPEAN PATENT APPLICATION**
published in accordance with Art. 153(4) EPC

(43) Date of publication:
**19.01.2022 Bulletin 2022/03**

(21) Application number: **20764925.2**

(22) Date of filing: **12.02.2020**

(51) International Patent Classification (IPC):
*A61P 9/00* (2006.01)    *C12N 5/071* (2010.01)
*C12N 5/0735* (2010.01)   *C12N 5/10* (2006.01)
*A61K 35/44* (2015.01)

(52) Cooperative Patent Classification (CPC):
**C12N 5/10;** A61K 35/44; A61P 9/00

(86) International application number:
**PCT/JP2020/005255**

(87) International publication number:
**WO 2020/179380 (10.09.2020 Gazette 2020/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.03.2019 JP 2019040117**

(71) Applicant: **Public University Corporation
Nagoya City University
Mizuho-ku
Nagoya-shi
Aichi 467-8601 (JP)**

(72) Inventors:
• **MATSUNAGA, Tamihide
Nagoya-shi, Aichi 467-8603 (JP)**
• **HASHITA, Tadahiro
Nagoya-shi, Aichi 467-8603 (JP)**
• **AOKI, Hiromasa
Nagoya-shi, Aichi 467-8603 (JP)**

(74) Representative: **Jaekel, Robert Sebastian
Witthoff Jaekel Steinecke, Patentanwälte PartG
mbB
Patent Department
P.O. Box 11 40
52412 Jülich (DE)**

(54) **PREPARATION AND EXPANSION CULTURE OF ENDOTHELIAL PROGENITOR CELL**

(57)    It is a subject to provide a means of preparing highly pure vascular endothelial progenitor cells in a simple and low-cost manner. It is also a subject to provide a method for efficiently proliferating vascular endothelial progenitor cells. High-purity vascular endothelial progenitor cells are prepared by the process of differentiating pluripotent stem cells into vascular endothelial progenitor cells and purifying the vascular endothelial progenitor cells using a difference in adhesion ability between the vascular endothelial progenitor cells constituting the cell population obtained in the process and other cells. On the other hand, vascular endothelial progenitor cells are cultured and expanded in the presence of a ROCK inhibitor, a GSK-3β inhibitor, and a TGF-β receptor inhibitor in addition to basic fibroblast growth factor and epidermal growth factor.

**Description**

[Technical Field]

The present invention relates to preparation and extended culture of vascular endothelial progenitor cells. Specifically, the present invention relates to a method for preparing high-purity vascular endothelial progenitor cells from pluripotent stem cells, a method for efficiently proliferating vascular endothelial progenitor cells, and the like. The present application claims priority based on Japanese Patent Application No. 2019-040117 filed on March 6, 2019, the contents of which are incorporated herein by reference in its entirety.

[Background Art]

Human pluripotent stem cells, including induced pluripotent stem cells (iPS cells) and embryonic stem cells (ES cells), have an ability to proliferate indefinitely and differentiate into all parts of the human body. Due to this property, human pluripotent stem cells (particularly, iPS cells) are expected to be used for construction of a drug screening model that mimics a human organ, clinical application in regenerative medicine, and the like.

Various clinical applications of iPS cell-derived vascular endothelial progenitor cells (EPCs) are conceivable, including regeneration of blood vessels, treatment of myocardial infarction by co-transplantation with cardiac muscle, and advanced three-dimensional cell tissue construction of organs. In addition, the cells are also expected to be used in a screening system in the construction of a pathological model of a vascular disease and in the development of new drugs by differentiation of the cells into brain capillary vascular endothelial cells. High-purity vascular endothelial progenitor cells are required in a large amount for use for such purposes.

Currently, there are many reports that vascular endothelial cells (ECs) and vascular endothelial progenitor cells are efficiently induced to differentiate from ES cells and iPS cells (see, for example, NPL 1 and NPL 2). However, since a rate of differentiation into vascular endothelial cells and vascular endothelial progenitor cells is greatly affected by the cell line used and their state before differentiation, purification is often performed finally using antibody beads/magnetic beads or a cell sorter in order to obtain high-purity cells. In addition, there are limited reports on efficient extended culture methods for iPS cell-derived vascular endothelial progenitor cells. The research group of the present inventors reported a novel vascular endothelial progenitor cell differentiation inducing method to which the iPS-sac method is applied in the previous patent application (PTL 1). Further, PTL 2 discloses a method for preparing vascular endothelial progenitor cells from embryonic stem cells.

[Citation List]

[Patent Literature]

[PTL 1]: JP 2018-110548 A
[PTL 1] WO 2008/056779

[Non Patent Literature]

[NPTL 1]: Mien T.X. et al. Differentiation of Human Embryonic stem cells to endothelial progenitor cells on laminins in defined and xeno-free systems. Stem cell reports, 2016;7:802-16.
[PTL 2]: Kyung-dal Chot. Et al. Hematopoietic and endothelial differentiation of human induced pluripotent stem cells. Stem cells, 2009;27:559-67.

[Summary of Invention]

[Technical Problem]

High-purity cells can be achieved according to a purification method using antibody beads/magnetic beads or a cell sorter, but cells are greatly damaged (for example, cells are exhausted due to a long treatment time). In addition, the operation for the method is complicated and requires a high cost. Furthermore, the possibility of contamination by xenogeneic components such as antibody-derived components cannot be excluded. In view of these circumstances, a first object of the present invention is to provide a means for preparing high-purity vascular endothelial progenitor cells simply and at low cost. Another object of the present invention is to enable a Xeno-free preparation process for vascular endothelial progenitor cells (preparation of vascular endothelial progenitor cells under conditions free of xenogeneic components). Still another object of the present invention is to provide a method for efficiently proliferating vascular endothelial progenitor cells.

[Solution to Problem]

When iPS cells are induced to differentiate into vascular endothelial progenitor cells, vascular endothelial progenitor cells and other unnecessary cells (e.g., cells different in differentiation direction) are usually mixedly present. Focusing on convenience, the present inventors have studied, from various angles, methods and conditions for selectively removing unnecessary cells in the culture process (in other words, selectively allowing vascular endothelial progenitor cells to remain in the culture system). As a result, it has been found by chance that there is a difference in adhesion ability between vascular endothelial progenitor cells and unnecessary cells, and the present inventors have succeeded in selectively removing unnecessary cells and increasing purity of vascular endothelial progenitor cells by mediating a specific detaching treatment. That is, the present inventors have established a method for selectively isolating vascular endothelial progenitor cells at a terminal stage of differentiation by utilizing a difference in adhesion ability between vascular endothelial progenitor cells and other cells. This method achieved high purity of vascular endothelial progenitor cells, comparable to that obtained by conventional methods, despite extremely simple manipulation. In addition, by using this method, selection by antibody beads/magnetic beads, a cell sorter, or the like becomes unnecessary, so that damage to cells and contamination by xenogeneic components (antibody-derived components and the like) can be avoided. It should be noted that the method was also applicable to vascular endothelial progenitor cells induced to differentiate under xenogeneic component-free (Xeno-free) conditions. This fact means that a technique enabling all the steps in the process from establishment of iPS cells to creation of vascular endothelial progenitor cells to be performed in a Xeno-free manner, that is, a technique extremely useful for practical use or clinical application has been successfully established.

On the other hand, it has been found that, when three specific low molecular weight compounds (ROCK inhibitor, GSK3β inhibitor, and TGFβ receptor inhibitor) are used in combination, a proliferation rate of iPS cell-derived vascular endothelial progenitor cells is dramatically increased. That is, the present inventors have succeeded in finding a "novel combination of low molecular weight compounds" that can greatly contribute to stable supply of vascular endothelial progenitor cells. This combination is also effective when vascular endothelial progenitor cells are cultured under Xeno-free conditions, and clinical application of the combination can be greatly expected.

[1] A method for preparing vascular endothelial progenitor cells, including the following steps (1) and (2):

(1) a step of differentiating pluripotent stem cells into vascular endothelial progenitor cells; and
(2) a step of purifying the vascular endothelial progenitor cells using a difference in adhesion ability between the vascular endothelial progenitor cells constituting a cell population obtained in step (1) and other cells,

[2] The preparation method according to [1], wherein step (2) includes the following steps (2-1) and (2-2):

(2-1) a step of detaching and removing the other cells from a culture surface by a first detaching treatment using a first cell dissociation solution; and
(2-2) a step of detaching the vascular endothelial progenitor cells from a culture surface by a second detaching treatment using a second cell dissociation solution, the second detaching treatment having a higher cell detaching effect than that of the first detaching treatment, and collecting the vascular endothelial progenitor cells.

[3] The preparation method according to [2], wherein the first detaching treatment and the second detaching treatment satisfy one or more of the following conditions (a) to (d) that:

(a) the second detaching treatment has a longer treatment time than that of the first detaching treatment;
(b) the second detaching treatment has a higher treatment temperature than that of the first detaching treatment;
(c) the second cell dissociation solution used in the second detaching treatment has a higher active ingredient concentration than that of the first cell dissociation solution used in the first detaching treatment; and
(d) the second cell dissociation solution used for the second detaching treatment has a stronger action of the active ingredient than that of the first cell dissociation solution used for the first detaching treatment.

[4] The preparation method according to [2] or [3], wherein the first detaching treatment includes contact of the cell population obtained in step (1) with the first cell dissociation solution and subsequent tapping treatment.

[5] The preparation method according to any one of [1] to [4], wherein step (1) consists of the following steps (1-1) and (1-2):

(1-1) a step of differentiating pluripotent stem cells into mesoderm; and
(1-2) a step of differentiating the cells obtained in step (1-1) into vascular endothelial progenitor cells.

[6] The preparation method according to [5], wherein a culture period in step (1-2) is 2 days to 14 days.

[7] The preparation method according to [5] or [6], wherein, in the middle of step (1-2), a culture surface having strong cell adhesion is switched to a culture surface having weak cell adhesion.

[8] The preparation method according to [5] or [6], wherein step (1-2) includes the following culturing steps (1-2-1) and (1-2-2): (1-2-1) culture in the presence of bone morphogenetic protein 4 and vascular endothelial growth factor; and
(1-2-2) culture in the presence of basic fibroblast growth factor and vascular endothelial growth factor.

[9] The preparation method according to [8], wherein a culture period in step (1-2-1) is 1 day to 7 days, and a culture period in step (1-2-2) is 1 day to 7 days.

[10] The preparation method according to [9], wherein

a culture surface having strong cell adhesion is used for the culture in step (1-2-1), and

a culture surface having weak cell adhesion is used for the culture in step (1-2-2).

[11] The preparation method according to [7] or [10], wherein the culture surface having strong cell adhesion is a culture surface coated with a basement membrane component or a fragment thereof, and the culture surface having weak cell adhesion is a culture surface coated with a material selected from the group consisting of gelatin, poly-D-lysine, and poly-L-lysine.

[12] The preparation method according to any one of [1] to [11], wherein all the steps are performed under conditions free of a xenogeneic animal-derived component.

[13] The preparation method according to any one of [1] to [12], wherein the pluripotent stem cells are induced pluripotent stem cells.

[14] The preparation method according to [13], wherein the induced pluripotent stem cells are human induced pluripotent stem cells.

[15] A vascular endothelial progenitor cell obtained by the preparation method according to any one of [1] to [14].

[16] The vascular endothelial progenitor cell according to [15], which has a purity of greater than or equal to 90%.

[17] A cell preparation including the vascular endothelial progenitor cell according to [15] or [16].

[18] Use of the vascular endothelial progenitor cell according to [15] or [16] for construction of a blood vessel in vitro or construction of a human blood-brain barrier model.

[19] An extended culture method for vascular endothelial progenitor cells, including a step of culturing vascular endothelial progenitor cells in the presence of a ROCK inhibitor, a GSK-3$\beta$ inhibitor, and a TGF-$\beta$ receptor inhibitor, in addition to basic fibroblast growth factor and epidermal growth factor.

[20] The extended culture method according to [19], wherein the ROCK inhibitor is Y-27632, the GSK-3$\beta$ inhibitor is CHIR 99021, and the TGF-$\beta$ receptor inhibitor is A 83-01.

[21] The extended culture method according to [19] or [20], wherein the vascular endothelial progenitor cells are cells obtained by inducing differentiation of pluripotent stem cells.

[22] The extended culture method according to [21], wherein the pluripotent stem cells are induced pluripotent stem cells.

[23] The extended culture method according to [22], wherein the induced pluripotent stem cells are human induced pluripotent stem cells.

[24] The extended culture method according to [19] or [20], wherein the vascular endothelial progenitor cells are cells prepared by the preparation method according to any one of [1] to [14].

[25] The extended culture method according to [19] or [20], wherein the vascular endothelial progenitor cells are vascular endothelial progenitor cells collected in advance from a living body or cells obtained by maintaining or proliferating the vascular endothelial progenitor cells in vitro.

[26] The extended culture method according to any one of [19] to [25], wherein the step is performed under conditions free of a xenogeneic animal-derived component.

[Brief Description of Drawings]

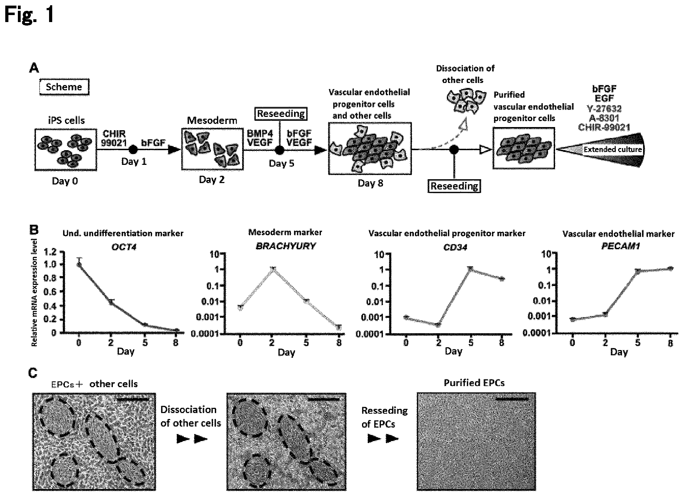

[Fig. 1] A: Vascular endothelial progenitor cell differentiation protocol. B: Analysis of gene expression levels before differentiation and on Day 2, Day 5, and Day 8 of differentiation. [Day 0: Human iPS cells (610B1 strain) mean ± S.D. (n = 3)]. C: Purification process of vascular endothelial progenitor cells (phase difference image) [Scale bar: 500 μm].

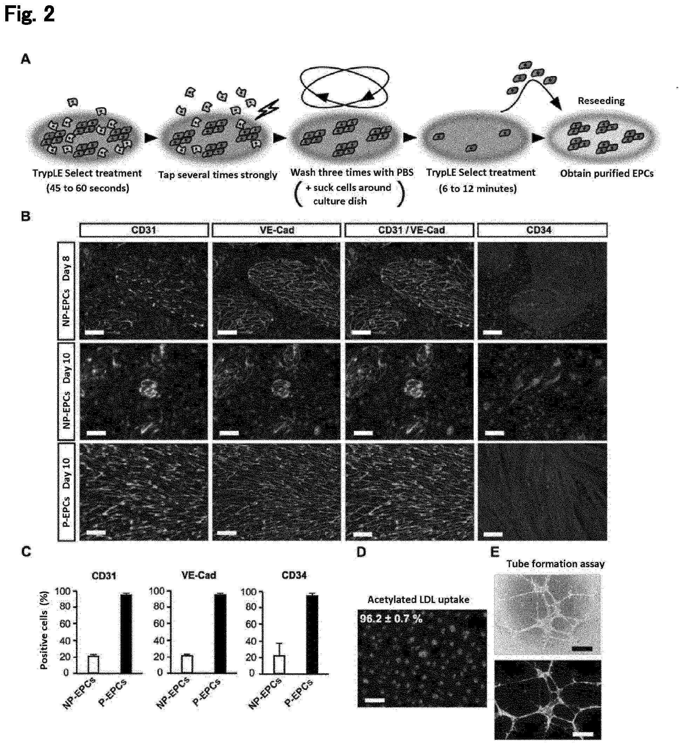

[Fig. 2] A: Overview of a method for purifying vascular endothelial progenitor cells. B: Immunostaining images of CD31, VE-Cadherin, and CD34 in NP-EPCs (non-purified vascular endothelial progenitor cells) and P-EPCs (purified vascular endothelial progenitor cells) [Scale bar: 100 μm].C: Numbers of positive cells (%) for CD31, VE-Cadherin, and CD34 in NP-EPCs and P-EPCs (Day 10) [Mean ± S.D. (n = 3)]. D: DiI-acetylated LDL or Hoechst 33342 was taken up by cells, images were acquired by Operetta high content imaging system, and a positive cell rate was calculated by analysis software [Scale bar: 100 μm] [Mean ± S.D. (n = 3)]. E: Images 20 hours after P-EPCs on Day 8 were seeded on a matrigel-coated culture dish; upper: phase difference image; lower: calcein [Scale bar: 500 μm].

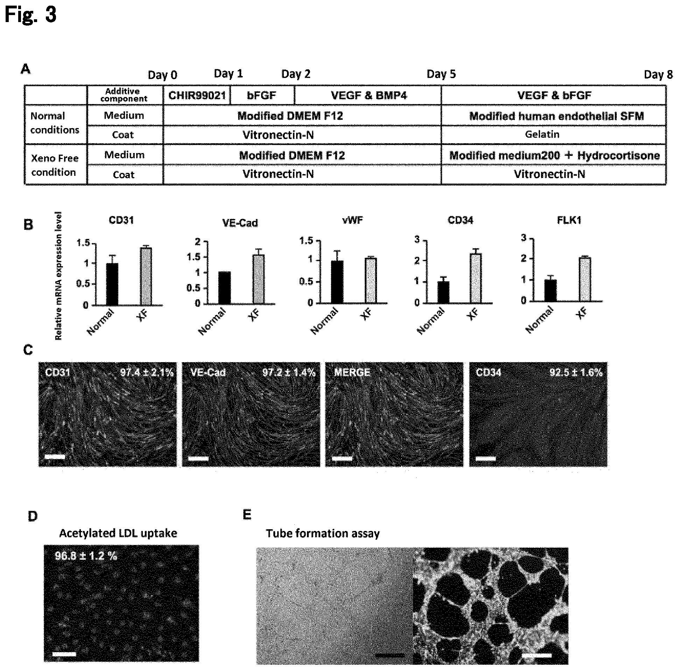

[Fig. 3] A: Differentiation induction protocol under Xeno-free conditions. B: Analysis of gene expression levels [Normal: Normal protocol (Day 8), XF: Xeno-Free protocol (Day 8), Mean ± S.D. (n = 3)]. C: Immunostaining images of CD31, VE-Cadherin, and CD34 in Xeno-free EPCs (Day 10) after selection and positive cell rates [Scale bar: 100 μm, Mean ± S.D. (n = 3)]. D: DiI-acetylated LDL or Hoechst 33342 was taken up by cells, images were acquired by Operetta high content imaging system, and a positive cell rate was calculated by analysis software [Scale bar: 100 μm]
[Mean ± S.D. (n = 3)]
E: Images 20 hours after XF-EPCs on Day 8 were seeded on a matrigel-coated culture dish. Left: Phase difference image, right: Calcein [Scale bar: 500 μm].

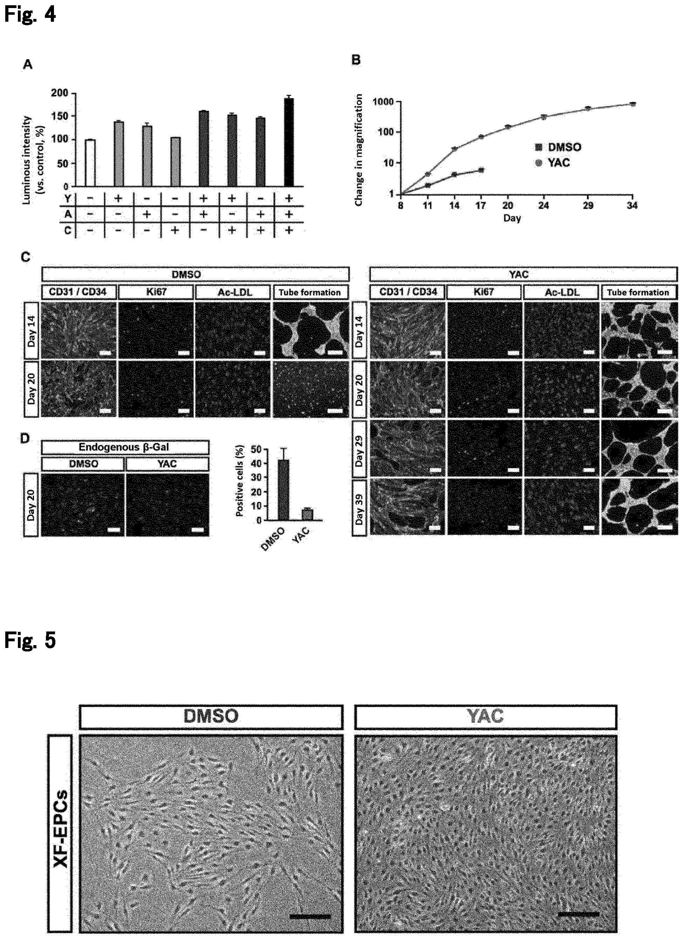

[Fig. 4] A: Cell proliferation potency from Day 11 to Day 14 was evaluated by Cell Titer Glo 2.0 assay. DMSO group = 100% [Mean ± S.D. (n = 6)]. B: Cell proliferation rates [Mean ± S.D. (n = 3)] of DMSO group and YAC group. C: Immunostaining of CD31, CD34, and Ki 67 at each passage in DMSO group and YAC group [Scale bar: 100 μm], uptake of AC-LDL [Scale bar: 100 μm], and tube formation [Scale bar: 500 μm]. D: Ratio of β-galactosidase-positive cells in DMSO group and YAC group [Scale bar: 500 μm].
[Fig. 5] Phase difference images of XF-EPCs in DMSO group and YAC group on Day 11.

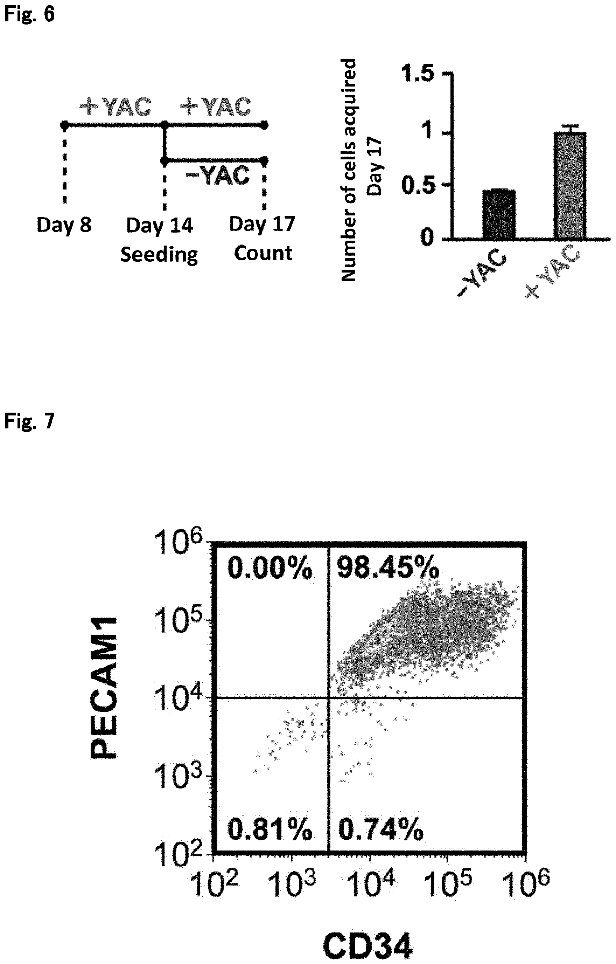

[Fig. 6] Number of cells acquired (+ YAC = 1) on Day 17.
[Fig. 7] Analysis of purity by flow cytometry.

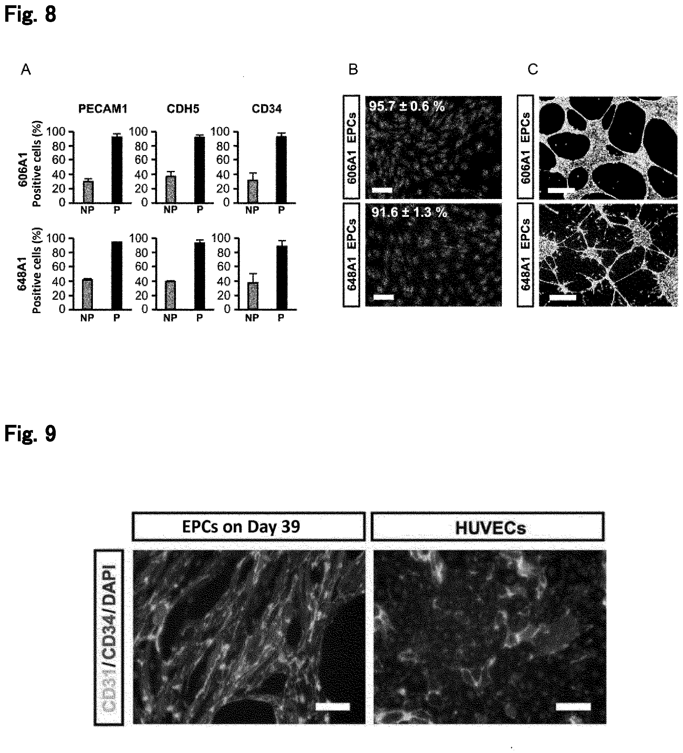

[Fig. 8] A: Numbers (%) of positive cells for PECAM1, CDH5 and CD34 in the differentiated cells (P) not subjected to purification step and the differentiated cells (NP) subjected to the purification step, on Day 10 of differentiation, derived from 606A1 and 648A1 strains [Mean ± S.D. (n = 3; ** p < 0.01)]. B: DiI-acetylated LDL or Hoechst 3334 was taken up by EPCs derived from 606A1 strain and 648A1 strain on Day 10 of differentiation, images were acquired by Operetta high content imaging system, and positive cell rate was calculated by analysis software [Scale bar: 100 μm] [Mean ± S.D. (n = 3)]. C: Images (calcein) 20 hours after purified EPCs derived from 606A1 and 648A1 strains on Day 8 of differentiation were seeded on a matrigel-coated culture dish [Scale bar: 500 μm].

[Fig. 9] Protein expression analysis of CD34 in EPCs and HUVECs. Protein expression of CD34 was compared between EPCs (YAC group, Day 39 of differentiation) and HUVECs. [Scale bar: 100 $\mu$m].

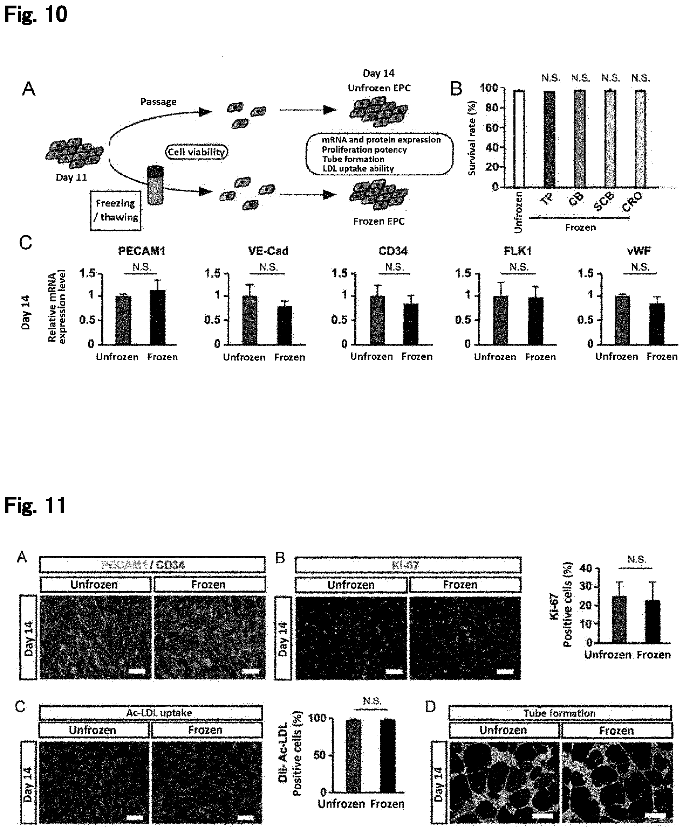

[Fig. 10] Analysis of influence of freezing and thawing on EPCs. A: Protocol for comparing the properties of unfrozen and frozen cells. B: Cell viabilities of unfrozen cells and cells frozen by slow freezing method were measured by trypan blue method and compared [TP: TC Protector, CB: Cell Banker, SCB: Stem Cell Banker, CRO: Cell Reservoir One, [Mean $\pm$ S.D. (n = 3; N.S. = no significant difference, Tukey's HSD test, unfrozen cells vs others]. C: Analysis of gene expression levels in unfrozen cells and frozen cells [Unfrozen cells = 1, mean $\pm$ S.D. (n = 3); N.S. = no significant difference, Student's t-test].

[Fig. 11] Analysis of influence of freezing and thawing on EPCs. A: Immunostaining images of PECAM1, CDH5, and CD34 in unfrozen cells and frozen cells [Scale bar: 100 $\mu$m]. B: Immunostaining images of Ki-67 in unfrozen and frozen cells and positive cell rates [Scale bar: 100 $\mu$m, mean $\pm$ S.D. (n = 3; N.S. = not significant, Student's t-test)]. C: Uptake of Dil-acetylated LDL in unfrozen cells and frozen cells and positive cell rates [Hoechst 33342 = blue, scale bar: 100 $\mu$m, mean $\pm$ S.D. (n = 3; N.S. = no significant difference, Student's t-test)]. D: Tube formation with unfrozen cells and frozen cells [Scale bar: 500 $\mu$m].

[Description of Embodiments]

1. Method for preparing vascular endothelial progenitor cells

A first aspect of the present invention relates to a method for preparing vascular endothelial progenitor cells (EPCs) from pluripotent stem cells (hereinafter, also referred to as "the preparation method of the present invention"). According to the present invention, vascular endothelial progenitor cells capable of differentiating into vascular endothelial cells (ECs) are obtained. When vascular endothelial progenitor cells are induced to differentiate under appropriate conditions, or vascular endothelial progenitor cells are placed in an environment suitable for induction of differentiation (typically, transplantation/administration into/to a site where vascular endothelial cells are present or vicinities thereof in a living body), vascular endothelial cells are produced. Vascular endothelial progenitor cells are used in regenerative therapies for severe ischemic diseases such as coronary artery disease and lower limb ischemic disease, vascular regeneration experiments, and the like. The preparation method of the present invention makes it possible to easily and inexpensively prepare such highly useful vascular endothelial progenitor cells.

The "pluripotent stem cell" refers to a cell having both the ability to differentiate into all cells constituting a living body (differentiation pluripotency) and the ability to produce daughter cells having the same differentiation potency as that of its own through cell division (self-replication potency). Differentiation pluripotency can be evaluated by transplanting cells to be evaluated into nude mice, and testing the presence or absence of teratoma formation containing cells of each of three germ layers (ectoderm, mesoderm, and endoderm).

Examples of the pluripotent stem cells include embryonic stem cells (ES cells), embryonic germ cells (EG cells), and induced pluripotent stem cells (iPS cells), but are not limited thereto as long as the pluripotent stem cells have both differentiation pluripotency and self-replication potency. Preferably, ES cells or iPS cells are used. Further preferably, iPS cells are used. The pluripotent stem cells are preferably mammalian (for example, primates such as humans and chimpanzees, and rodents such as mice and rats) cells, particularly preferably human cells. Therefore, in the most preferred embodiment of the present invention, human iPS cells are used as the pluripotent stem cells.

ES cells can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press(1994); Thomson, J. A. et al., Science,282, 1145-1147(1998)). As the initial embryo, an early embryo produced by nuclear transfer of a nucleus of a somatic cell may be used (Wilmut et al. (Nature, 385, 810(1997)), Cibelli et al. (Science, 280, 1256(1998)), Akira Iriya et al. (Protein Nucleic Acid and Enzyme, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000), Tachibana et al. (Human Embryonic Stem Cells Derived by Somatic Cell Nuclear Transfer, Cell (2013) in press). A parthenogenetic embryo may be used as an early embryo (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). Besides the above articles, as for the production of ES cells, reference can be made to Strelchenko N., et al. Reprod Biomed Online. 9: 623-629, 2004; Klimanskaya I., et al. Nature 444: 481-485, 2006; Chung Y., et al. Cell Stem Cell 2: 113-117, 2008; Zhang X., et al Stem Cells 24: 2669-2676, 2006; Wassarman, P.M. et al. Methods in Enzymology, Vol. 365, 2003, and the like. Fused ES cells obtained by cell fusion of ES cells and somatic cells are also included in the embryonic stem cells used in the method of the present invention.

Some ES cells are available from preservation institutions or are commercially available. For example, human ES cells are available from the Institute for Frontier Medical Sciences, Kyoto University (for example, KhES-1, KhES-2 and KhES-3), WiCell Research Institute, ESI BIO, and the like.

EG cells can be established, for example, by culturing primordial germ cells in the presence of LIF, bFGF, or SCF (Matsui et al., Cell, 70, 841-847 (1992), Shamblott et al., Proc. Natl. Acad. Sci. USA, 95 (23), 13726-13731 (1998), Turnpenny et al., Stem Cells, 21(5), 598-609, (2003)).

The "induced pluripotent stem cell (iPS cell)" is a cell having pluripotency (multipotency) and proliferation potency, which is produced by reprogramming a somatic cell by introduction of an initialization factor or the like. The induced pluripotent stem cells exhibit properties close to those of ES cells. The somatic cell used in the production of an iPS cell is not particularly limited, and may be a differentiated somatic cell or an undifferentiated stem cell. Also, its origin is not particularly limited, but somatic cells of mammals (for example, primates such as humans and chimpanzees, and rodents such as mice and rats) are preferably used, and human somatic cells are particularly preferably used. iPS cells can be produced by various methods reported so far. In addition, the application of an iPS cell preparation method which will be developed in the future is naturally contemplated.

The most fundamental technique of iPS cell preparation methods is a method of introducing four factors, Oct 3/4, Sox2, Klf4, and c-Myc, which are transcription factors, into cells using viruses (Takahashi K, Yamanaka S: Cell 126 (4), 663-676, 2006; Takahashi, K, et al: Cell 131 (5), 861-72, 2007). There is a report on establishment of human iPS cells by introduction of four factors Oct4, Sox2, Lin 28, and Nonog (Yu J, et al: Science 318(5858), 1917-1920, 2007). The establishment of iPS cells by introduction of the three factors other than c-Myc (Nakagawa M, et al: Nat.Biotechnol. 26 (1), 101-106, 2008), two factors Oct 3/4 and Klf (Kim J B, et al: Nature 454 (7204), 646-650, 2008), or only Oct 3/4 (Kim J B, et al: Cell 136 (3), 411-419, 2009) has also been reported. In addition, a method of introducing a protein, which is an expression product of a gene, into a cell (Zhou H, Wu S, Joo JY, et al: Cell Stem Cell 4, 381-384, 2009; Kim D, Kim CH, Moon JI, et al: Cell Stem Cell 4, 472-476, 2009) has also been reported. On the other hand, it has also been reported that the use of an inhibitor BIX-01294 against histone methyl transferase G9a, a histone deacetylase inhibitor valproic acid (VPA), BayK 8644, or the like makes it possible, for example, to improve the production efficiency and to reduce the factor to be introduced (Huangfu D, et al: Nat. Biotechnol. 26 (7), 795-797, 2008; Huangfu D, et al: Nat. Biotechnol. 26 (11), 1269-1275, 2008; Silva J, et al: PLoS. Biol. 6 (10), e 253, 2008). Studies have also been advanced on gene transfer methods, and techniques utilizing not only retroviruses, but also lentiviruses (Yu J, et al: Science 318(5858), 1917-1920, 2007), adenoviruses (Stadtfeld M, et al: Science 322 (5903), 945-949, 2008), plasmids (Okita K, et al: Science 322 (5903), 949-953, 2008), transposon vectors (Woltjen K, Michael IP, Mohseni P, et al: Nature 458, 766-770, 2009; Kaji K, Norrby K, Pac a, et al: Nature 458, 771-775, 2009; Yusa K, Rad R, Takeda J, et al: Nat Methods 6, 363-369, 2009), or episomal vectors (Yu J, Hu K, Smuga-Otto K, Tian S, et al: Science 324, 797-801, 2009) have been developed.

Cells that have been transformed into iPS cells, that is, initialized (reprogrammed) can be selected by using, as an index, expression of pluripotent stem cell markers (undifferentiation markers) such as Fbxo15, Nanog, Oct/4, Fgf-4, Esg-1, and Cript. The selected cells are collected as iPS cells.

iPS cells can also be provided, for example, the National University Corporation Kyoto University or the National Research and Development Agency, RIKEN BioResource Center.

In the present specification, "differentiate" refers to causing a cell to differentiate along a specific cell lineage, that is, inducing differentiation. In the preparation method of the present invention, after inducing differentiation of pluripotent stem cells into vascular endothelial progenitor cells, the vascular endothelial progenitor cells are purified. Specifically, in the preparation method of the present invention, the following steps (1) and (2) are performed:

> (1) a step of differentiating pluripotent stem cells into vascular endothelial progenitor cells; and
> (2) a step of purifying the vascular endothelial progenitor cells using a difference in adhesion ability between vascular endothelial progenitor cells constituting the cell population, which are obtained in step (1), and other cells.

<Step (1): Induction of differentiation into vascular endothelial progenitor cells>

In this step, pluripotent stem cells are cultured and differentiated into vascular endothelial progenitor cells. In other words, pluripotent stem cells are cultured under conditions for inducing differentiation into vascular endothelial progenitor cells. Culture conditions are not particularly limited as long as the pluripotent stem cells differentiate into vascular endothelial progenitor cells. Typically, two-step differentiation induction which will be described below, that is, a step of differentiating the pluripotent stem cells into mesoderm (step (1-1)) and a step of differentiating the obtained cells into vascular endothelial progenitor cells (step (1-2)) are performed so that the pluripotent stem cells are differentiated into vascular endothelial progenitor cells via mesoderm.

Step (1-1): Differentiation into mesoderm

In this step, pluripotent stem cells are cultured and differentiated into mesoderm. In other words, pluripotent stem cells are cultured under conditions for inducing differentiation into mesoderm. Culture conditions are not particularly limited as long as the pluripotent stem cells differentiate into mesoderm. For example, in accordance with a previous report (for example, reference can be made to Sriram G. et al, Efficient differentiation of human embryonic stem cells to arterial and venous endothelial cells under feeder- and serum-free conditions. Stem Cell Research & Therapy 2015; 6:261), the cells are cultured in the presence of a GSK-3β inhibitor, and then cultured in the presence of basic fibroblast growth factor (bFGF).

The phrase "in the presence of a GSK-3β inhibitor" is synonymous with the condition that the GSK-3β inhibitor is added to the medium. Therefore, in order to culture the cells in the presence of the GSK-3β inhibitor, a medium to which the GSK-3β inhibitor is added may be used. Examples of the GSK-3β inhibitor may include CHIR 99021, SB216763, CHIR 98014, TWS119, Tideglusib, SB415286, BIO, AZD2858, AZD1080, AR-A014418, TDZD-8, LY2090314, IM-12, Indirubin, Bikinin, and 1-Azakenpaullone. An examplary concentration of the GSK-3β inhibitor to be added (in the case of CHIR 99021) is 1 μM to 100 μM, preferably 3 μM to 30 μM. A period of culture (culture period) in the presence of the GSK-3β inhibitor is, for example, 1 day to 4 days, preferably 1 day to 2 days. When a compound different from the exemplified compound, that is, CHIR 99021, is used, those skilled in the art can set the concentration of the compound to be added in accordance with the above concentration range, in consideration of differences in properties (particularly, a difference in activity) between the compound to be used and the exemplified compound. Whether or not the set concentration range is appropriate can be confirmed by a preliminary experiment according to Examples which will be described later.

The phrase "in the presence of basic fibroblast growth factor (bFGF)" is synonymous with the condition that bFGF is added to the medium. Therefore, in order to culture the cells in the presence of bFGF, a medium to which bFGF is added may be used. An exemplary concentration of bFGF to be added is 5 ng/mL to 500 ng/mL, preferably 10 ng/mL to 200 ng/mL. As the bFGF, human bFGF (for example, human recombinant bFGF) is preferably used. The bFGF is also called fibroblast growth factor 2 (FGF2).

Step (1-2): Differentiation into vascular endothelial progenitor cells

In this step, the cells obtained in step (1-1) are cultured and differentiated into vascular endothelial progenitor cells. In other words, the cells are cultured under conditions for inducing differentiation into vascular endothelial progenitor cells. Culture conditions are not particularly limited as long as differentiation into vascular endothelial progenitor cells can be induced. For example, the differentiation into vascular endothelial progenitor cells is induced using bone morphogenetic protein 4 (BMP4), vascular endothelial growth factor (VEGF), bFGF, or the like as a differentiation-inducing factor.

In order to enhance the efficiency, operability, and the like of a detaching treatment in the next step, that is, step (2) (purification of vascular endothelial progenitor cells), it is preferable to switch from a culture surface having strong cell adhesion to a culture surface having weak cell adhesion in the middle of step (1-2). In other words, at the end of step (1-2), the cells may be brought into a state of being cultured on a culture surface having weak cell adhesion. According to this embodiment, it is easy to detach unnecessary cells in step (2), that is, cells other than the vascular endothelial progenitor cells.

For example, a culture surface formed by coating a surface of a culture vessel (for example, a culture dish, a culture flask, or a microplate for cell culture) with one or more of basement membrane components (for example, laminin (laminin 511 or the like), collagen (type IV collagen or the like) 4, entactin, vitronectin, or fibronectin) or a fragment thereof (for example, an E8 fragment of vitronectin-N or laminin (laminin 511 or the like)) and the like can be employed as the "culture surface having strong cell adhesion". As described above, the "culture surface having strong cell adhesion" can be configured using a substance exhibiting high adhesive force to the cells. The "culture surface having weak cell adhesion" is a culture surface having weak cell adhesion (that is, having relatively weak cell adhesion) as compared with the "culture surface having strong cell adhesion", and is a culture surface having cell adhesion similarly to the "culture surface having strong cell adhesion". The "culture surface having weak cell adhesion" can be configured by, for example, coating a surface of the culture vessel with one or more of gelatin, poly-D-lysine, poly-L-lysine, and the like. The level of cell adhesion may be increased or decreased by a difference in concentration of the surface coating agent (density of the cell adhesive substance to be present on the culture surface).

A period (culture period) of step (1-2) is, for example, 2 days to 14 days, preferably 4 days to 10 days.

In a preferred embodiment, as step (1-2), two-step culture, that is, culture in the presence of BMP4 and VEGF (step (1-2-1)), and culture in the presence of bFGF and VEGF (step (1-2-2)), which is performed after the culture in step (1-2-1), are performed. According to this embodiment, it is possible to form a state of high cell density before the next step (step (2)) while inducing efficient differentiation into vascular endothelial progenitor cells. It is preferable to increase the cell density, in view of increasing the difference in adhesion ability between the vascular endothelial progenitor cells and the other cells, and it is advantageous for selective detachment of the cells other than the vascular endothelial progenitor cells.

The phrase "in the presence of BMP4 and VEGF" in step (1-2-1) is synonymous with the condition that BMP4 and VEGF are added to the medium. Therefore, in order to culture the cells in the presence of BMP4 and VEGF, a medium to which BMP4 and VEGF are added may be used. BMP4 is a differentiation-inducing factor and, when used, allows the cells to differentiate into vascular endothelial progenitor cells. A concentration of BMP4 to be added is, for example, 5 mg/mL to 200 ng/mL, preferably 10 mg/mL to 100 mg/mL. On the other hand, a concentration of VEGF as a blood vessel inducing factor is, for example, 10 ng/mL to 200 ng/mL, preferably 20 ng/mL to 100 ng/mL. Preferably, human BMP4 (for example, human recombinant BMP4) and human VEGF (for example, human recombinant VEGF) are used.

A period (culture period) of step (1-2-1) is, for example, 1 day to 7 days, preferably 2 days to 5 days. If the culture period is too short, the expected effect (that is, induction of differentiation into vascular endothelial progenitor cells) cannot be sufficiently obtained. On the other hand, when the culture period is too long, induction of differentiation into different cells is caused.

The phrase "in the presence of bFGF and VEGF" in step (1-2-2) is synonymous with the condition that bFGF and VEGF are added to the medium. Therefore, in order to culture the cells in the presence of bFGF and VEGF, a medium to which bFGF and VEGF are added may be used. A concentration of bFGF to be added is, for example, 1 ng/mL to 5200 ng/mL, preferably 2 ng/mL to 100 ng/mL. A concentration of VEGF to be added is, for example, 10 ng/mL to 200 ng/mL, preferably 20 ng/mL to 100 ng/mL. As in the case of the above steps, human bFGF (for example, human recombinant bFGF) and human VEGF (for example, human recombinant VEGF) are preferably used.

A period (culture period) of step (1-2-2) is, for example, 1 day to 7 days, preferably 2 days to 5 days. If the culture period is too short, the expected effect (that is, proliferating the cells while inducing differentiation into vascular endothelial progenitor cells) cannot be sufficiently obtained. On the other hand, when the culture period is too long, induction of differentiation into different cells is caused.

In a preferred embodiment, the switching of the culture surface, that is, the switching from the "culture surface having strong cell adhesion" to the "culture surface having weak cell adhesion" is synchronized with the switching from step (1-2-1) to step (1-2-2). In other words, the "culture surface having strong cell adhesion" is used for the culture in step (1-2-1), and the "culture surface having weak cell adhesion" is used for the culture in step (1-2-2). According to this embodiment, the operation is simplified, and cell damage can be minimized.

In each step (step (1), step (1-1), step (1-2), step (1-2-1), and step (1-2-2)) constituting the present invention, subculture may be performed in the middle. For example, when the cells become confluent or sub-confluent, some of the cells are collected and transferred to another culture vessel, and the culture is continued. It is preferable to set the cell density low in order to promote differentiation. For example, the cells may be seeded at a cell density of about $1 \times 10^4$ cells/cm$^2$ to $1 \times 10^6$ cells/cm$^2$. For collection of the cells, a cell dissociation solution or the like may be used. As the cell dissociation solution, for example, proteolytic enzymes such as trypsin-EDTA, various collagenases (for example, collagenase IV), metalloprotease and dispase can be used alone or in appropriate combination. In addition, commercially available cell dissociation solutions such as TrypLE™ (Invitrogen), Acutase™ (Innova Cell Technologies), and Accumax™ (Innova Cell Technologies) may be used.

At the time of collection of cells associated with medium replacement, subculture, and the like, the cells may be treated in advance with a ROCK inhibitor (Rho-associated coiled-coil forming kinase/Rho-binding kinase) such as Y-27632 in order to suppress cell death.

Other culture conditions (such as culture temperature) in each step constituting the present invention may be conditions generally employed in culture of animal cells. That is, for example, the culture may be performed in an environment of 37°C and 5% $CO_2$. As a basal medium, for example, Iscove's Modified Dulbecco's Medium (IMDM) (GIBCO), MEMα medium, serum-free medium for vascular endothelial cells (Human Endothelial SFM (Gibco), Medium 200 (Gibco), etc.), Ham's F12 medium (HamF12) (SIGMA, Gibco, etc.), Dulbecco's Modified Eagle's Medium (D-MEM) (Nacalai Tesque, Sigma, Gibco, etc.), Glasgow's basal medium (Gibco, Inc), RPMI 1640 medium, MCDB 107 medium (Research Institute for the Functional Peptides), or the like can be used. A medium in which two or more basal media are mixed, for example, a mixed medium of D-MEM and Ham's F12 medium may be used.

Examples of other components that can be added to the medium can include serum (such as fetal bovine serum, human serum, and sheep serum), serum replacements (such as Knockout serum replacement (KSR)), antibiotics (such as penicillin and streptomycin), supplements (such as ITS-G supplement), L-glutamine, L-ascorbic acid phosphate magnesium salt n-hydrates, non-essential amino acids (NEAA), 2-mercaptoethanol, and Chemically Defined Lipid Concentrate (Gibco).

In a preferred embodiment, step (1) is carried out under Xeno-free conditions. When step (1) is composed of step (1-1) and step (1-2), these two steps are performed under Xeno-free conditions. Similarly, when step (1-2) is composed of step (1-2-1) and step (1-2-2), these two steps are also performed under Xeno-free conditions. In the case of culturing the cells under Xeno-free conditions, a serum-free medium is usually employed, and KnockOut™ SR XenoFree Medium (Gibco), ITS-G, hormones (human recombinant hydrocortisone and the like), Chemically Defined Lipid Concentrate and the like are preferably added for the purpose of improving the cell proliferation rate and the like.

Differentiation into vascular endothelial progenitor cells can be determined or evaluated by using, for example, expression of a vascular endothelial progenitor cell marker as an index. Examples of the vascular endothelial progenitor cell marker include PECAM1 (CD31), CD34, CDH5 (VE-Cadherin), and FLK1 (VEGFR-2). Among them, CD34 is vascular endothelial progenitor cell-specific and is a particularly useful vascular endothelial progenitor cell marker.

On the other hand, if a function as a vascular endothelial cell is shown when differentiation into vascular endothelial cells has been induced, it can be confirmed that the cells have been differentiated into vascular endothelial progenitor cells. For evaluating a function as a vascular endothelial cell, a Dil-Ac LDL uptake test and a tube formation test can be used.

<Step (2): Purification of vascular endothelial progenitor cells>

A cell population containing vascular endothelial progenitor cells is obtained by step (1). In step (2) following step (1), vascular endothelial progenitor cells are purified. The phrase "purification of vascular endothelial progenitor cells" refers to increasing the ratio of vascular endothelial progenitor cells constituting a cell population (that is, abundance ratio or content rate). Therefore, if the ratio of vascular endothelial progenitor cells is higher after a treatment than that before the treatment, the treatment corresponds to purification. A degree of purification (purity) is not particularly limited. The purity can vary depending on the state of cells to be used, culture conditions, and the like, but, for example, a purity of 80% or more, preferably a purity of 90% or more, more preferably a purity of 95% or more can be achieved through step (2). The purity can be calculated by the following calculation formula. For measurement of the number of cells, a hemocytometer or the like may be used.

$$\text{Purity (\%)} = (\text{Number of vascular endothelial progenitor cells})/(\text{Number of all cells constituting cell population}) \times 100$$

Step (2) is one of the greatest features of the present invention, in which the vascular endothelial progenitor cells are purified using a difference in adhesion ability between the vascular endothelial progenitor cells constituting a cell population obtained in step (1) and other cells, based on the finding that the vascular endothelial progenitor cells and the other cells are difference in adhesive force to the culture surface. As shown in Examples which will be described below, the vascular endothelial progenitor cells exhibit higher adhesion ability than the other cells. Therefore, the vascular endothelial progenitor cells are typically purified by preferentially or selectively detaching and removing the other cells having low adhesion ability (the vascular endothelial progenitor cells are selectively maintained in the culture system). In this case, for example, the following steps (2-1) and (2-2) may be performed:

(2-1) a step of detaching and removing the other cells from the culture surface by a first detaching treatment using a first cell dissociation solution; and
(2-2) a step of detaching the vascular endothelial progenitor cells from a culture surface and collecting the vascular endothelial progenitor cells by a second detaching treatment using a second cell dissociation solution, the second detaching treatment having a higher cell detaching effect than that of the first detaching treatment.

Step (2-1) is a process of selectively removing unnecessary cells, that is, cells other than vascular endothelial progenitor cells. After this treatment, the remaining cells are collected by step (2-2) to obtain a cell population including the vascular endothelial progenitor cells in high purity.

In the first detaching treatment in step (2-1), typically, the cell population obtained in step (1) is brought into contact with the first detaching solution to weaken the adhesive force between the cells and the culture surface (a state in which the cells are easily detached is formed), and then an impact by tapping, pipetting or the like is applied to dissociate unnecessary cells to be removed, that is, cells other than vascular endothelial progenitor cells, from the culture surface. The dissociated cells are removed, and the next step, that is, step (2-2) is performed. "Tapping" refers to giving an intermittent and abrupt impact to the contents by, for example, flicking a wall surface or the like of the culture vessel with a finger, a rod, or the like, or gripping an upper portion or the like of the culture vessel and striking the culture vessel against the wall surface or the like with a light snap.

In step (2-2) following step (2-1), the remaining cells, that is, vascular endothelial progenitor cells are detached from the culture surface and collected by the same operation using the second cell dissociation solution. The contact of the cell dissociation solution with the cell population is not usually instantaneous, and the contact state is maintained for a certain period of time.

In order to achieve high purity of vascular endothelial progenitor cells by step (2-1) and step (2-2), for example, conditions for the first detaching treatment in step (2-1) and the second detaching treatment in step (2-2) are set so as to satisfy one or more of the following conditions (a) to (d). As active ingredients of the first cell dissociation solution and the second cell dissociation solution, proteolytic enzymes such as trypsin-EDTA, various collagenases (for example, collagenase IV), metalloprotease and dispase can be used alone or in appropriate combination. In addition, commercially available cell dissociation solutions such as TrypLE™ (Invitrogen), Acutase™ (Innova Cell Technologies), and Accumax™ (Innova Cell Technologies) may be used.

(a) The second detaching treatment has a longer treatment time than that of the first detaching treatment.
(b) The second detaching treatment has a higher treatment temperature than that of the first detaching treatment.
(c) The second cell dissociation solution used in the second detaching treatment has a higher active ingredient concentration than that of the first cell dissociation solution used in the first detaching treatment.
(d) The second cell dissociation solution used for the second detaching treatment has a stronger action of the active ingredient than that of the first cell dissociation solution used for the first detaching treatment.

For conditions (a) to (c), usually, the first cell dissociation solution and the second cell dissociation solution containing the same active ingredient are prepared, and the first detaching treatment and the second detaching treatment are performed. Therefore, it is easy to prepare the cell dissociation solutions. In this regard, under conditions (a) and (b), cell dissociation solutions having the same composition including the active ingredient can be utilized, which is particularly advantageous. In addition, condition (a) only needs to provide a difference in treatment time between the first detaching treatment and the second detaching treatment, is simpler than condition (b) that provides a difference in treatment temperature, and can be said to be an optimum condition.

On the other hand, condition (c) and condition (d) are conditions using the second cell dissociation solution having a higher detaching effect than that of the first cell dissociation solution, and, in the case where these conditions are adopted, two types of cell dissociation solutions are prepared when the concentration of the active ingredient is different (in the case of condition (c)) or the active ingredient itself is different (in the case of condition (d)).

The optimum conditions for the first detaching treatment and the second detaching treatment may vary depending on the type and state of the cells to be used, the active ingredients of the cell dissociation solutions, or other factors, but may be set by a preliminary experiment with reference to the disclosure of the present specification. Hereinafter, examples of the active ingredients and concentrations of the cell dissociation solutions, and the treatment times and treatment temperatures of the detaching treatments will be described.

Active ingredient of cell dissociation solution: EDTA, trypsin-EDTA, various collagenases, metalloprotease, dispase

Active ingredient concentration of cell dissociation solution: EDTA: 0.5 mM to 500 mM, Trypsin-EDTA: 0.005 to 0.5%, Collagenase IV: 0.005 to 0.5%, Dispase: 100 to 2000 PU/mL
Treatment time of detaching treatment: 5 seconds to 1 hour
Treatment temperature of detaching treatment: 10°C to 38°C

In the case of condition (a), for example, the treatment time of the second detaching treatment is set to 1.5 to 10 times the treatment time of the first detaching treatment. In the case of condition (b), for example, the first detaching treatment is performed at 20°C to 30°C, and the second detaching treatment is performed at 30°C to 38°C. In the case of condition (c), for example, the active ingredient concentration of the second cell dissociation solution is set to 1.5 to 10 times the active ingredient concentration of the first cell detaching solution. For condition (d), for example, the active ingredient of the first cell dissociation solution is set to collagenase or EDTA (two or more of them may be combined), and the active ingredient of the second cell detaching solution is set to trypsin-EDTA or dispase (two or more of them may be combined).

When a commercially available cell dissociation solution such as TrypLE™ (Invitrogen), Acutase™ (Innova Cell Technologies), or Accumax™ (Innova Cell Technologies) is used, the first detaching treatment and the second detaching treatment can be more easily performed. In this case, any one of conditions (a) to (c), more preferably condition (a) or (c), or still more preferably condition (a) are/is adopted in consideration of reduction of the type/number of cell dissociation solutions to be used, simplicity of operation, and the like.

The purified cells (high-purity vascular endothelial progenitor cells) are used for various applications. In addition, the cells are subjected to culture for maintenance and proliferation as necessary. Alternatively, the cells are preserved until use (the subjecting the cells to culture also corresponds to the use referred to herein).

For the culture for maintenance and proliferation, various culture methods (for example, James D. et al, Expansion and maintenance of human embryonic stem cell-derived endothelial cells by TGFbeta inhibition is Id1 dependent. Nature Biotechnology. 2010;28(2):161-6. and Mien T.X. et al. Differentiation of Human Embryonic stem cells to endothelial progenitor cells on laminins in defined and xeno-free systems. Stem cell reports. 2016;7:802-16.) can be used as long as they are suitable for culturing vascular endothelial progenitor cells. In a preferred embodiment, the cells after purification are cultured by the extended culture method of the present invention which will be described below.

A preservation method used when the purified cells are preserved may follow a conventional method. For example, TC protector (DS Pharma Biomedical Co., Ltd), Cell Banker (Zenoac Co., Ltd), Stem Cell Banker (Zenoac Co., Ltd), Cell Reserver One (Nacalai Co., Ltd), and the like are used for cryopreservation.

2. Use of vascular endothelial progenitor cells

A second aspect of the present invention relates to use of a vascular endothelial progenitor cell obtained by the preparation method of the present invention (for convenience of description, hereinafter, referred to as "the vascular endothelial progenitor cell of the present invention"). As a first use, a cell preparation containing the vascular endothelial progenitor cell of the present invention is provided. As described above, the vascular endothelial progenitor cell is expected to be applied to treatment of coronary artery disease, lower limb ischemic disease (e.g., Buerger's disease and arteriosclerosis obliterans), and the like. In fact, clinical trials using vascular endothelial progenitor cells have also been conducted, and good results have been reported. The cell preparation of the present invention can be used for treatment (regenerative medicine) of various diseases in which administration/transplantation of vascular endothelial progenitor cells is effective.

The cell preparation of the present invention can be prepared, for example, by suspending the vascular endothelial progenitor cells of the present invention in physiological saline, a buffer (for example, a phosphate buffer), or the like. For example, $1 \times 10^5$ to $1 \times 10^{10}$ cells may be contained as a single dose so that a therapeutically effective amount of the cells can be administered. The content of the cells can be appropriately adjusted in consideration of the purpose of use, the target disease, the sex, age, and body weight of a subject (recipient) to be applied with the cell preparation, the condition of an affected area, condition of the cells, and the like.

Dimethyl sulfoxide (DMSO), serum albumin, and the like may be contained in the cell preparation of the present invention for the purpose of protecting cells, antibiotics and the like may be contained in the cell preparation for the purpose of preventing bacterial contamination, and various components (vitamins, cytokines, growth factors, steroids, etc.) may be contained in the cell preparation for the purpose of activating the cells, proliferating the cells, inducing differentiation of the cells, and the like. In addition, other pharmaceutically acceptable components (for example, carriers, excipients, disintegrants, buffers, emulsifiers, suspension agents, soothing agents, stabilizers, preservatives, antiseptics, physiological saline, etc.) can be contained in the cell preparation of the present invention.

Instead of being used for regenerative medicine as a cell preparation, the vascular endothelial progenitor cell of the present invention can also be used for constructing a material for use in regenerative medicine, specifically, a blood vessel for transplantation in vitro. The method for constructing a blood vessel using vascular endothelial progenitor cells is detailed in, for example, Revascularization Therapy (supervised by Hiroo Imura, Shindan To Chiryo Sha, Inc.).

It is also possible to utilize the vascular endothelial progenitor cell of the present invention for construction of a human blood-brain barrier model. For example, vascular endothelial progenitor cells are seeded in an insert part of Transwell, pericytes are seeded on a back side thereof, and astrocytes are seeded on a bottom surface of the well, thereby mimicking the same structure as the blood-brain barrier in a living body (Nakagawa S, Maria A. D, Nakao S, Honda M, Hayashi M, Nakaoke R, Kataoka Y, Niwa M. Cellular and Molecular Neurobiology. 27:687-694, 2007).

3. Extended culture of vascular endothelial progenitor cells

A third aspect of the present invention relates to an extended culture method for vascular endothelial progenitor cells. The "extended culture" refers to increasing the number of cells while maintaining the properties of the cells. When vascular endothelial progenitor cells are subjected to extended culture, the vascular endothelial progenitor cells can be expanded while maintaining their properties (in particular, differentiation ability into vascular endothelial cells). According to the extended culture method of the present invention, a high growth rate is obtained, and efficient extended culture can be performed. Therefore, the present invention is useful as a means for preparing a large amount of vascular endothelial progenitor cells useful for clinical use, research use, and the like, and contributes to stable supply of vascular endothelial progenitor cells.

In the extended culture method of the present invention, a characteristic step of "culturing vascular endothelial progenitor cells in the presence of a ROCK inhibitor, a GSK-3β inhibitor, and a TGF-β receptor inhibitor, in addition to basic fibroblast growth factor (bFGF) and epidermal growth factor (EGF)" is performed. The phrase "in the presence of a ROCK inhibitor, a GSK-3β inhibitor, and a TGF-β receptor inhibitor, in addition to basic fibroblast growth factor (bFGF) and epidermal growth factor (EGF)" has the same meaning as the condition that bFGF, EGF, ROCK inhibitor, GSK-3β inhibitor, and TGF-β receptor inhibitor are added to the medium. The former two (bFGF and EGF) are factors generally used in culturing vascular endothelial progenitor cells and vascular endothelial cells. A concentration of bFGF to be added is, for example, 5 ng/mL to 200 ng/mL, preferably 10 ng/mL to 50 ng/mL, and a concentration of EGF to be added is, for example, 5 ng/mL to 500 ng/mL, preferably 10 ng/mL to 200 ng/mL. On the other hand, the latter three (ROCK inhibitor, GSK-3β inhibitor, and TGF-β receptor inhibitor) are characteristic of the present invention, that is, the use of a combination of these three compounds constitutes the basis of the present invention.

Although not bound by theory, the ROCK inhibitor can be expected to have an effect of suppressing cell death and enhancing cell proliferation potency. In addition, the GSK-3β inhibitor has a wnt pathway activating action, and can be expected to have an effect of enhancing the cell proliferation potency via a wnt/β-catenin signal. The TGF-β receptor inhibitor can be expected to have an effect of enhancing cell proliferation potency by suppressing a TGFβ signal known as a cell growth inhibitory factor. The proliferation potency of vascular endothelial progenitor cells is dramatically improved by a synergistic effect obtained by using these three compounds in combination.

For example, Y-27632 can be used as the ROCK inhibitor. On the other hand, examples of the GSK-3β inhibitor may include CHIR 99021, SB216763, CHIR 98014, TWS119, Tideglusib, SB415286, BIO, AZD2858, AZD1080, AR-A014418, TDZD-8, LY2090314, IM-12, Indirubin, Bikinin, and 1-Azakenpaullone. Examples of the TGF-β receptor inhibitors are A 83-01, SB431542, SB-505124, SB525334, D4476, ALK5 inhibitor, LY2157299, LY364947, GW788388, and RepSox.

An exemplary concentration of the ROCK inhibitor to be added (in the case of Y-27632) is 1 μM to 50 μM, preferably 3 μM to 30 μM. An exemplary concentration of the GSK-3β inhibitor to be added (in the case of CHIR 99021) is 0.1 μM to 100 μM, preferably 1 μM to 30 μM. An exemplary concentration of the TGF-β receptor inhibitor to be added (in the case of A 83-01) is 0.05 μM to 20 μM, preferably 0.1 μM to 10 μM.

When compounds different from the exemplified compounds, that is, Y-27632, CHIR99021 and A 83-01, are used, those skilled in the art can set the concentrations of the compounds to be added in accordance with the above concentration ranges, in consideration of differences in properties (particularly, a difference in activity) between the compounds to be used and the exemplified compounds. Whether or not the set concentration range is appropriate can be confirmed by a preliminary experiment according to Examples which will be described later.

Other culture conditions may be set according to a conventional method (for example, reference can be made to reports such as Ikuno T. et al, Efficient and robust differentiation of endothelial cells from human induced pluripotent stem cells via lineage control with VEGF and cyclic AMP.PLOS ONE. 2017; 12: 3; and Li S. et al, Inhibition of cell growth and induction of inflammation by endosulfan in HUVEC-C cells. Environmental Toxicology. 2016; 12: 1785-1795), that is, culture may be performed using a general medium (see the description of the medium in the first aspect) under an environment of, for example, 37°C and 5% $CO_2$.

Examples of other components that can be added to the medium can include serum (such as fetal bovine serum, human serum, and sheep serum), serum replacements (such as Knockout serum replacement (KSR)), antibiotics (such as penicillin and streptomycin), supplements (such as ITS-G supplement), L-glutamine, L-ascorbic acid phosphate magnesium salt n-hydrates, non-essential amino acids (NEAA), 2-mercaptoethanol, and Chemically Defined Lipid Concentrate.

In a preferred embodiment, the following steps are carried out under Xeno-free conditions. In the case of culturing the cells under Xeno-free conditions, a serum-free medium is usually employed, and KnockOut™ SR XenoFree Medium, hormones (human recombinant hydrocortisone and the like), ITS-G, Chemically Defined Lipid Concentrate and the like are preferably added for the purpose of improving the cell proliferation rate and the like.

Passaging is performed as necessary. Usually, when the cells become confluent or sub-confluent, the cells are passaged to further proliferate and maintain the cells. The subculture operation and the like are the same as those in the first aspect, and the detailed description thereof will be omitted.

The vascular endothelial progenitor cells to be subjected to the extended culture method of the present invention are not particularly limited. In addition to vascular endothelial progenitor cells obtained by inducing differentiation of pluripotent stem cells, the extended culture method of the present invention can be applied to vascular endothelial progenitor cells collected in advance from a living body or cells obtained by maintaining or proliferating the vascular endothelial progenitor cells in vitro (cells immediately after collection, cells preserved after collection, cells maintenance-cultured after collection, cells cultured after collection and proliferated, etc.).

The differentiation inducing method in the case of using vascular endothelial progenitor cells obtained by inducing differentiation of pluripotent stem cells is not particularly limited. Therefore, vascular endothelial progenitor cells prepared by a differentiation inducing method which will be developed in the future as well as the previously reported differentiation inducing methods (see, for example, reports such as Lian X. et al, Efficient differentiation of human pluripotent stem cells to endothelial progenitors via small-molecule activation of WNT signaling. Stem cell reports. 2014; 3 (5): 804-16.; and Mien T.X. et al. Differentiation of Human Embryonic stem cells to endothelial progenitor cells on laminins in defined and xeno-free systems. Stem cell reports, 2016; 7: 802-16) can be subjected to the extended culture method of the present invention. The "pluripotent stem cells" are as defined in the description of the preparation method of the present invention, and embryonic stem cells (ES cells), embryonic germ cells (EG cells), induced pluripotent stem cells (iPS cells), and the like correspond to the pluripotent stem cells. As the pluripotent stem cells, ES cells or iPS cells are preferably used, and iPS cells (particularly preferably, human iPS cells) are more preferably used.

As a preferred embodiment, the extended culture method of the present invention is applied to the vascular endothelial progenitor cell obtained by the preparation method of the present invention. That is, high-purity vascular endothelial progenitor cells derived from pluripotent stem cells prepared through a characteristic purification step are subjected to extended culture to obtain high-purity vascular endothelial progenitor cells suitable for various applications (particularly, medical applications).

When vascular endothelial progenitor cells collected in advance from a living body are used, the living body is typically a human. For example, vascular endothelial progenitor cells derived from a patient with a disease for which a therapeutic effect by transplantation/transfer of vascular endothelial progenitor cells is expected, for example, coronary artery disease or lower limb ischemic disease (e.g., Buerger's disease or arteriosclerosis obliterans) can be subjected to the extended culture method of the present invention.

The cells after extended culture are used for various applications in the same manner as the cells obtained by the preparation method of the present invention (see item 2 above). In the case of not being used immediately, the cells may be preserved. The description concerning the first aspect is incorporated, as to the method for preserving cells.

[Examples]

In order to develop a method for purifying vascular endothelial progenitor cells induced to differentiate from pluripotent stem cells, the following experiments were conducted. In addition, the extended culture of vascular endothelial progenitor cells was studied.

1. Method

(1) Coating of culture surface

Gelatin: A 0.1% gelatin solution was allowed to stand at 37°C for 1 hour or at 4°C overnight.
Vitronectin-N (VTN-N): Diluted with D-PBS (-) so as to attain 1 $\mu$g/cm$^2$, and allowed to stand at 37°C for 1 hour to 2 hours.
Fibronectin: Diluted with D-PBS (-) so as to be 1 $\mu$g/cm$^2$, and allowed to stand at 37°C for 1 hour or at 4°C overnight.

(2) Culture of human living body-derived cells

Human umbilical vein endothelial cells (HUVECs) were purchased from ScienCell and cultured using ECM medium (ScienCell). Passaging was performed once every 3 days, and medium exchange was performed on Day 2 after passaging. In addition, a fibronectin-coated culture dish was used for culture. A number of cells to be seeded was 5,000 cells/cm$^2$.

(3) Culture of human iPS cells

An experiment was performed using a 610B1 strain (established at Kyoto University) established by introducing 5 factors (Oct 3/4, Sox2, Klf4, L-Myc, and Lin 28) into human cord blood using an episomal vector (pCXLE). The 610B1 strain was cultured using Essential 8 flex medium (Gibco) at 37°C in a CO$^2$ incubator under 5% CO$^2$/95% air conditions. As a detaching solution at the time of passage of the 610B1 strain, 0.5M EDTA (pH 8.0) or TrypLE™ Select (Gibco) was used. When TrypLE™ Select was used for detachment, 10 $\mu$M Y-27632 was added immediately after passage, and the medium was replaced on the next day. In addition, culture was performed on a culture dish coated with VTN-N.

(4) Differentiation of human iPS cells into vascular endothelial progenitor cells

The cells were seeded on the VTN-N-coated culture dish at a density of 2 $\times$ 10$^4$ cells/cm$^2$ and cultured in Essential 8 Flex medium for 24 hours. Thereafter, the cells were cultured for 1 day in a medium in which 5 $\mu$M CHIR 99021 was added to modified DMEM/F12 (450 mmol/L monothioglycerol, 50 $\mu$g/mL L-ascorbic acid phosphate magnesium salt n-hydrate, 1/10 ITS, 2 mM Gluta-MAX, 0.1% chemically defined concentrate, 1% Penicillin-Streptomycin). Thereafter, the cells were cultured for 1 day in a medium in which 50 ng/mL bFGF was added to modified DMEM/F12. Thereafter, the cells were cultured for 3 days in a medium in which 50 ng/mL VEGF and 25 ng/mL BMP4 were added to modified DMEM/F12. During this procedure, the medium was replaced every day. On Day 5, cells treated with 10 $\mu$M Y-27632 for 1 hour were dissociated with TrypL™E Select (Gibco), and passaged in a culture dish coated with new gelatin at 3.5 $\times$ 10$^4$ cells/cm$^2$, and differentiation induction was performed in a medium in which 50 ng/mL VEGF and 10 ng/mL bFGF were added to modified Human endothelial SFM {Human Endothelial SFM (Gibco) + 5% knockout serum replacement (Gibco)} or a medium in which 2.5 μM Hydrocortisone, 50 ng/mL VEGF and 10 ng/mL bFGF were added to modified medium 200 {medium 200 (Gibco) + 5% knockout serum replacement XenoFree Medium (Gibco) + 1/10 ITS}. During the procedure, the medium was replaced every day.

(5) Purification of EPCs

The cells on Day 8 of differentiation treated with 10 μM Y-27632 for 1 hour were treated with TrypLE™ Select (Gibco) for 45 to 60 seconds, and cells other than EPCs (Extra cells) were detached to some extent, and Extra cells were completely detached by hitting (tapping) the culture dish with several flicks. At this time, when the cells at the end of the culture dish were hardly detached, the cells at the end of the culture dish were sucked so as to draw a circle. Thereafter, the cells were washed with PBS three times or more, treated again with TrypLE™ Select for 6 to 12 minutes to detach the EPCs, and then collected in a centrifuge tube and subjected to centrifugation operation (1000 rpm, 5 min) to be reseeded in a new culture dish.

(6) Culture of differentiated cells

The purified cells were cultured in a modified Human endothelial SFM supplemented with 10 ng/mL EGF, 20 ng/mL bFGF, and DMSO (DMSO group) or 10 μM Y-27632, 0.5 μM A 83-01, or 3 μM CHIR 99021 (YAC group). For culture under Xeno-free conditions, a medium prepared by adding DMSO or YAC to a medium prepared by adding 2.5 μM Hydrocortisone, 10 ng/mL EGF, and 20 ng/mL bFGF to modified medium 200 was used. Medium exchange was performed on Days 1 and 3 after seeding. In addition, passaging was performed at intervals of 3 to 5 days. At the time of passage, the cells were washed once with PBS, and then treated with TrypLE™ Select at 37°C for 5 minutes or longer to detach the cells. Thereafter, the cells were collected in a 15 mL tube or a 50 mL tube with the medium and centrifuged at 1,000 rpm for 5 minutes. Thereafter, the cells were resuspended using a medium, and reseeded at $1.5 \times 10^4$ cells/cm² in a culture dish coated with VTN-N. The number of cells was counted through trypan blue staining and automatically measured by Countess II (Invitrogen).

(7) Cell Titer Glo 2.0 Assay

The Cell Titer Glo 2.0 assay was performed according to the delivered manual. A luminous intensity was measured by Synergy HTX Gen5 with Gain set to an automatic mode. In addition, for the measurement, the cells on Day 11 of differentiation were seeded in a 48 well plate at $0.5 \times 10^3$ cells/cm², and cultured for 3 days.

(8) Angiogenesis assay (tube formation assay)

On Day 8 of differentiation, $7.5 \times 10^4$ (under normal conditions) or $1 \times 10^5$ (under Xeno-free conditions) endothelial cells selectively obtained were seeded on a 24 well plate coated with 300 μL Matrigel. A medium prepared by adding 50 ng/mL VEGF, 10 ng/mL EGF, and 20 ng/mL bFGF to modified Human Endothelial SFM (under normal conditions) or a medium prepared by adding 2.5 μM Hydrocortisone, 50 ng/mL VEGF, 10 ng/mL EGF, and 20 ng/mL bFGF to modified medium 200 (under Xeno-free conditions) was used. After a lapse of 20 hours, Calcein-AM (living cell observation reagent) having a final concentration of 2 μM was reacted for 30 minutes, and the cell morphology was observed with a microscope. The cells after Day 8 were seeded with $0.5 \times 10^5$ to $1 \times 10^5$ cells under the same conditions as described above. For culture for 20 hours, a medium prepared by newly adding 50 ng/mL VEGF to the medium (growth factors, including low molecular weight compounds) used for maintenance culture under each condition immediately before this culture was used.

(9) Immunostaining

The cells on a 96 well plate were fixed in 4% paraformaldehyde for 15 min at room temperature, washed twice with glycine-containing PBS, and then permeabilized with PBS containing 0.1% Triton x-100 for 25 min at room temperature. After blocking for 20 min at room temperature with 5% donkey serum, primary antibodies were reacted for 2h at room temperature. After washing three times with PBS, the secondary antibody was reacted at 200-fold dilution for 60 minutes at room temperature. At this time, DAPI as each staining reagent was also reacted at the same time (final concentration: 1 μg/mL). Thereafter, after washing with PBS three times, analysis was performed with Operetta high content imaging system (PerkinElmer). A ratio of CD31- and CD144-positive cells was automatically calculated by Operetta analysis system as the number of cells having a certain luminance or more/the total number of cells. An average number of positive cells in six visual fields in one well was calculated, and the calculation was performed for three wells (n = 3).

(10) Acetylated LDL uptake test

Acetylated LDL labeled with Dil was reacted with the cells on Day 10 of differentiation at a final concentration of 10 μg/mL for 5 hours, and then Hoechist 33342 was reacted for 30 minutes. The cells were washed four times with a medium, and analyzed by Operetta high content imaging system. A ratio of acetylated LDL-positive cells was automatically calculated by Operetta analysis system as the number of cells having a certain luminance or more/the total number of cells. An average number of positive cells in six visual fields in one well was calculated, and the calculation was performed for three wells (n = 3).

(11) Detection of β-galactosidase

β-galactosidase was detected using Cellular Senescence Detection Kit-SPiDER-βGal (Dojindo) according to the delivered manual. Cells were seeded in a 96 well plate, cultured for 3 days, then washed once with medium, and reacted for 1 hour in medium (50 μl) diluted 1000 fold with Barifomycin A1 working solution. Thereafter, a medium (50 μl) supplemented with SPiDER β-Gal working solution (1/1000 amount) and Hoechist 33342 (final concentration: 10 μg/mL) was added to each well, and the cells were cultured for 30 minutes. After that, the cells were washed twice with the medium and analyzed with Operetta high content imaging system. A ratio of β-galactosidase-positive cells was automatically calculated by Operetta analysis system as the number of cells having a certain luminance or more/the total number of cells. An average number of positive cells in six visual fields in one well was calculated, and the calculation was performed for three wells (n = 3).

(12) RNA extraction

Extraction was performed according to the attached manual of Agencourt (registered trademark) RNAdvance™ Tissue Kit.

(13) Reverse transcription reaction

Synthesis of complementary DNA (cDNA) was performed using ReverTra Ace (registered trademark) qPCR RT Master Mix according to the attached manual.

(14) RT-q PCR method

RT-q PCR was performed using KAPA SYBR Fast qPCR Kit using cDNA as a template, and the reaction was performed according to the attached manual. Results were corrected using HPRT as an endogenous control.
The characteristics of the marker genes used in this study are shown below.

PECAM1 (CD31): Representative vascular endothelial cell marker
CD34: Hematopoietic cells, vascular endothelial progenitor cell marker
CDH5 (VE-Cadherin): Adhesion molecule belonging to the cadherin family, expressed in vascular endothelial cells
vWF: Representative vascular endothelial cell marker
FLK1: Representative mesoderm to vascular endothelial cell marker
OCT4: Representative undifferentiation marker
BRACHYURY: Representative mesodermal marker

(15) Measurement of purity by flow cytometry
A 15 mL tube containing the medium in which the purified EPCs (Day 14 of differentiation) were suspended was centrifuged at 100 × g for 5 minutes, and the medium was sucked. Thereafter, fixation was performed with 1 mL of 4% paraformaldehyde for 10 minutes. Centrifugation was performed at 100 × g for 5 minutes, 4% paraformaldehyde was sucked, and the cells were permeabilized using cooled methanol at room temperature for 10 minutes. The fixed cells were centrifuged at 100 × g for 5 minutes, resuspended in 5 mL of Flow Cytometry Staining Buffer, and centrifuged at 100 × g for 5 minutes. After suction of Flow Cytometry Staining Buffer, a solution obtained by adding FITC-PECAM1 (10-fold dilution) and PE-CD34 (10-fold dilution) to 50 μL Flow Cytometry Staining Buffer was added, transferred to a 1.5 mL tube, and rotated at 4°C for 1 hour. Thereafter, the mixture was centrifuged at 100 × g at 4°C for 5 minutes, washed once with Flow Cytometry Staining Buffer, suspended in 500 μL of Flow Cytometry Staining Buffer, and analyzed by CytoFLEX (Beckman Coulter, Inc).
The following antibodies were used.

PE-CD34 antibody (Beckman Coulter, Inc. IM 1459 U)
FITC-PECAM1(Beckman Coulter, Inc. IM1431U)

(16) Freezing and thawing of cells

Freezing of differentiated cells was performed using TC Protector, CELLBANKER, STEM-CELLBANKER (containing DMSO) or Cell Reservoir One (containing DMSO). The mixture was resuspended in each preservation solution, and then frozen in a deep freezer at-80°C for 1 hour. At the time of thawing, the cell suspension was semi-thawed in a warm bath at 37°C, 1 mL was taken from 10 mL of the medium contained in a 15-mL tube or a 50-mL tube warmed in advance, and poured into the semi-thawed cell suspension to completely thaw the cell suspension. Then, the suspension was returned to the tube, and centrifuged at $100 \times g$ for 5 minutes. The medium was then sucked, and then the cells were suspended in a fresh medium and used for subsequent experiments. When the thawed cells were seeded, $2 \times 10^4$ cells/cm$^2$ were seeded.

2. Results and Considerations

(1) Induction of differentiation into EPCs

First, based on the past report (Sriram G. et al, Efficient differentiation of human embryonic stem cells to arterial and venous endothelial cells under feeder- and serum-free conditions, Stem Cell Research & Therapy. 2015;6:261), differentiation into mesoderm was induced by treatment with CHIR 99021 at 5 μM until Day 1 and treatment with bFGF at 50 ng/ml until Day 2, and the cell differentiation was directed to EPCs with BMP4 at 25 ng/mL and VEGF at 50 ng/mL in a period from Day 2 to Day 5 of differentiation (Fig. 1A). Modified DMEM/F12 was used as a medium.

Thereafter, in a period from Day 5 to Day 8 of differentiation, the cells were induced to differentiate into more mature EPCs in modified human endothelial SFM supplemented with 10 ng/mL bFGF and 50 ng/mL VEGF. When gene expression levels of the cells before differentiation (iPSCs) and on Days 2, 5, and 8 of differentiation were analyzed by RT-qPCR, the expression level of OCT4 as an undifferentiation marker decreased over time, and that of BRACHYURY as a mesoderm marker peaked on Day 2 of differentiation. In addition, the expression level of CD34 as a vascular endothelial progenitor cell marker peaked on Day 5, and that of PECAM1 (CD31) as a mature vascular endothelial cell marker remarkably increased on Days 5 and 8. Thus, it was confirmed that the induction of differentiation into EPCs proceeded by this protocol (Fig. 1B).

Two populations, which were clearly different in morphology, were observed on Day 8 of differentiation. One was a spindle-shaped cell which is a characteristic of a typical endothelial cell, and the other cell (Extra cell) clearly different therefrom in terms of morphology (Fig. 1C). Taking advantage of the difference in adhesion ability between the two cell groups, we developed a technique in which only Extra cells are detached first, and then EPCs are detached and passaged (Fig. 1C and Fig. 2A).

(2) Purification of vascular endothelial progenitor cells

The cells were treated with TrypLE™ Select at 37°C for 45 to 60 second. When the extra cells were detached, the culture dish was tapped several times to completely detach the extra cells. Thereafter, the cells were washed three or more times with PBS, and the adhered spindle-shped cells were detached again with TrypLE™ Select and seeded in a new culture dish (Fig. 2A).

Subsequently, protein expression of the cells on Day 8 and Day 10 of differentiation (Non Purified EPCs: NP-EPCs) and the purified cells (Purified EPCs: P-EPCs) was examined by immunostaining, and localization of CD31 and VE-Cadherin on the cell membranes was confirmed. Furthermore, the expression of CD34, as the vascular endothelial progenitor cell marker, was also confirmed. In addition, in the NP-EPCs, there were cells (Extra cells) not expressing CD31, VE-Cadherin, or CD34. However, the entire cells after selection expressed the vascular endothelial (precursor) cell marker. Thus, it was confirmed that the P-EPCs have higher purity than the NP-EPCs (Fig. 2C). Subsequently, in order to confirm how much the cells were actually purified, the numbers of positive cells for CD31, VE-Cadherin, and CD34 were calculated using the expression of CD31, CD144, and CD34 as indexes. As a result, the P-EPCs showed much higher values than the NP-EPCs. The purity of the P-EPCs was estimated to be 90% or higher. On the other hand, as a result of confirming the purity of the purified cells (P-EPCs) on Day 14 of differentiation by flow cytometry, the purity was 98.45%. That is, extremely high-purity cells were obtained (Fig. 7).

Subsequently, in order to confirm whether the P-EPCs had a function as vascular endothelial cells, an acetylated LDL uptake test was performed. As a result, most of the cells retained the ability to uptake acetylated LDL (Fig. 2E). Subsequently, when a tube formation assay using Matrigel was performed, a blood-vessel-like structure was observed (Fig. 2E). From the above, it was confirmed that the P-EPCs have a function as vascular endothelial cells.

On the other hand, also in the case of EPCs differentiated from other iPS cells (606A1 strain and 648A1 strain) by the same method, as a result of comparison in protein expression between when the EPCs were purified (P) and when the EPCs were not purified (NP), it was confirmed that high-purity cells were obtained by purification (Fig. 8A), supporting high versatility. In addition, the EPCs derived from the 606A1 strain and the EPCs derived from the 648A1 strain retained the ability to uptake acetylated LDL (Fig. 8B), exhibited the ability to form a blood-vessel-like structure in the tube formation assay (Fig. 8C), and exerted a function as vascular endothelial cells.

(3) Production of high-purity EPCs under Xeno-free conditions

Subsequently, since application of vascular endothelial progenitor cells to regenerative medicine is expected, differentiation induction under xenogeneic component-free (Xeno-free) conditions was attempted. Specifically, the medium used for culture from Day 5 to Day 8 was changed from modified human endothelial SFM to modified Medium 200 supplemented with hydrocortisone, and the coating agent was changed from gelatin to VTN-N (Fig. 3A). Under this condition, the XF-EPCs subjected to the purification operation on Day 8 of differentiation had almost the same expression levels of the vascular endothelial cell-related gene groups as compared with those in the case of normal protocol (Fig. 3B). Furthermore, expression of CD31, CD144, and CD34 was confirmed, and it was found that the purity was also high as in the case of normal protocol (Fig. 3C). The XF-EPCs also had the ability to uptake acetylated LDL (Fig. 3D) and the ability to form a tube (Fig. 3E) as functions of vascular endothelial cells. From the above, it was shown that vascular endothelial progenitor cells can differentiate into EPCs even under Xeno-free conditions, and that a purification method using a difference in adhesion ability between cell types is applicable.

(4) Establishment of extended culture method for iPS cell-derived EPCs

It was found that there is room for improvement in proliferation potency of the produced iPS cell-derived EPCs. Therefore, in order to improve the proliferation potency of EPCs, we focused on low molecular weight compounds that are inexpensive and easy to handle, and searched for an effective method for improving the proliferation potency of EPCs. We focused on a combination of three low molecular weight compounds (Y-27632 as a ROCK inhibitor, CHIR 99021 as a GSK3β inhibitor, and A 83-01 as a TGFβ inhibitor) allowing mouse hepatocytes to acquire undifferentiation properties and also enhancing the proliferation potency. There is a report that Y-27632 has a function of enhancing the proliferation potency of ECs derived from iPS cells, and that SB 43152, which is a compound similar to A-8301 among TGFβ inhibitors, enhances the proliferation potency of ECs derived from ES cells. Furthermore, there is a report that CHIR 99021 enhances the proliferation potency of HUVECs. Therefore, studies were conducted on all combinations of single groups, two-compound groups, and three-compound group (YAC group). As a result, EPCs in the YAC group showed the highest proliferation potency (Fig. 4A). It was confirmed that, when the DMSO group and the YAC group were maintenance-passaged, the YAC group could be passaged longer while maintaining the proliferation potency, and that the phenotype as a vascular endothelial progenitor cell was maintained (Fig. 4B and 4C). In addition, it was confirmed that cell senescence was less in the YAC group than in the DMSO group (Fig. 4D). Furthermore, the YAC group (Day 39 of differentiation) highly expressed the vascular endothelial progenitor cell marker CD34 as compared with HUVEC (Fig. 9).

(5) Effect of combination of Y-27632, A 83-01 and CHIR 99021 under Xeno-free conditions

When XF-EPCs after purification on Day 8 of differentiation were cultured for 3 days in the absence (DMSO group) or presence (YAC group) of YAC, the number of cells in the YAC group was clearly increased as compared with that in the DMSO group (Fig. 5). That is, the cell proliferation effect of YAC was obtained even under Xeno-free conditions.

(6) Examination of cell proliferation rate when Y-27632, A 83-01, and CHIR 99021 were removed from medium

When YAC was removed from the medium, the enhanced proliferation potency was reduced again (Fig. 6), and thus it was confirmed that EPCs stimulated by YAC were not cells that acquired the autonomous proliferation potency, which is a characteristic seen in cancer cells. Therefore, it is considered that EPCs proliferated by YAC can be used in regenerative medicine and the like.

(7) Influence of freezing and thawing on EPCs

The cells on Day 11 of differentiation were subjected to freezing-thawing treatment, and the influence of freezing and thawing on the cells was examined (Fig. 10A). The cells subjected to freezing-thawing treatment (frozen cells) and the cells not subjected to freezing treatment (unfrozen cells) showed equivalent survival rates (Fig. 10B), and there was no significant difference in expression of each marker between both cells (Fig. 10C). In addition, the results of immunostaining (Figs. 11A and 11B), the results of the acetylated LDL uptake test (Fig. 11C) and the tube formation assay (Fig. 11D) showed that the frozen cells and the unfrozen cells have equivalent functions as vascular endothelial cells.

3. Conclusion

In this study, by utilizing the difference in adhesion ability between human iPS cell-derived EPCs and other cells at the terminal stage of differentiation, high-purity EPCs could be easily and damagelessly isolated without using a cell sorter or magnetic beads. Furthermore, the proliferation potency of EPCs was successfully increased dramatically by adding a plurality of low molecular weight compounds in combination. In addition, the obtained EPCs retained their functions even after freezing-thawing treatment. This fact indicates that the cells can be cryopreserved in a state in which their properties are maintained, and is of great practical significance. This result is expected to greatly contribute to stable supply of human iPS cell-derived vascular endothelial progenitor cells contemplated to be used in various applications such as regenerative medicine.

[Industrial Applicability]

The preparation method of the present invention enables preparation of high-purity vascular endothelial progenitor cells despite simple manipulation. The high-purity vascular endothelial progenitor cells are useful for construction of a drug screening model, clinical application (for treatment of coronary artery disease, lower limb ischemic disease, and the like, construction of a blood vessel for regenerative medicine in vitro, and the like), and the like. The present invention is expected to be applied to these applications. On the other hand, the extended culture method of the present invention enables preparation of a large amount of vascular endothelial progenitor cells, and contributes to stable supply of vascular endothelial progenitor cells, for example. Both the preparation method and the extended culture method of the present invention are suitable for use under Xeno-free conditions, and in particular, are expected to greatly contribute to clinical applications of vascular endothelial progenitor cells (for example, regenerative medicine).
The present invention is not limited to the above embodiments and examples of the invention at all. Various modifications are also included in the present invention as long as they can be easily conceived by those skilled in the art without departing from the scope of the claims. The contents of the papers, published patent gazettes, patent gazettes, etc. clarified therein shall be incorporated by reference in their entirety.

**Claims**

1. A method for preparing vascular endothelial progenitor cells, comprising the following steps (1) and (2):

   (1) a step of differentiating pluripotent stem cells into vascular endothelial progenitor cells; and
   (2) a step of purifying the vascular endothelial progenitor cells using a difference in adhesion ability between the vascular endothelial progenitor cells constituting a cell population obtained in step (1) and other cells,

2. The preparation method according to claim 1, wherein step (2) comprises the following steps (2-1) and (2-2):

   (2-1) a step of detaching and removing the other cells from a culture surface by a first detaching treatment using a first cell dissociation solution; and
   (2-2) a step of detaching the vascular endothelial progenitor cells from a culture surface by a second detaching treatment using a second cell dissociation solution, the second detaching treatment having a higher cell detaching effect than that of the first detaching treatment, and collecting the vascular endothelial progenitor cells.

3. The preparation method according to claim 2, wherein the first detaching treatment and the second detaching treatment satisfy one or more of the following conditions (a) to (d) that:

   (a) the second detaching treatment has a longer treatment time than that of the first detaching treatment;
   (b) the second detaching treatment has a higher treatment temperature than that of the first detaching treatment;
   (c) the second cell dissociation solution used in the second detaching treatment has a higher active ingredient concentration than that of the first cell dissociation solution used in the first detaching treatment; and
   (d) the second cell dissociation solution used for the second detaching treatment has a stronger action of the active ingredient than that of the first cell dissociation solution used for the first detaching treatment.

4. The preparation method according to claim 2 or 3, wherein the first detaching treatment includes contact of the cell population obtained in step (1) with the first cell dissociation solution and subsequent tapping treatment.

5. The preparation method according to any one of claims 1 to 4, wherein step (1) consists of the following steps (1-1) and (1-2):

(1-1) a step of differentiating pluripotent stem cells into mesoderm; and
(1-2) a step of differentiating the cells obtained in step (1-1) into vascular endothelial progenitor cells.

6. The preparation method according to claim 5, wherein a culture period in step (1-2) is 2 days to 14 days.

7. The preparation method according to claim 5 or 6, wherein, in the middle of step (1-2), a culture surface having strong cell adhesion is switched to a culture surface having weak cell adhesion.

8. The preparation method according to claim 5 or 6, wherein step (1-2) comprises the following culturing steps (1-2-1) and (1-2-2): (1-2-1) culture in the presence of bone morphogenetic protein 4 and vascular endothelial growth factor; and
(1-2-2) culture in the presence of basic fibroblast growth factor and vascular endothelial growth factor.

9. The preparation method according to claim 8, wherein a culture period in step (1-2-1) is 1 day to 7 days, and a culture period in step (1-2-2) is 1 day to 7 days.

10. The preparation method according to claim 9, wherein

a culture surface having strong cell adhesion is used for the culture in step (1-2-1), and
a culture surface having weak cell adhesion is used for the culture in step (1-2-2).

11. The preparation method according to claim 7 or 10, wherein the culture surface having strong cell adhesion is a culture surface coated with a basement membrane component or a fragment thereof, and the culture surface having weak cell adhesion is a culture surface coated with a material selected from the group consisting of gelatin, poly-D-lysine, and poly-L-lysine.

12. The preparation method according to any one of claims 1 to 11, wherein all the steps are performed under conditions free of a xenogeneic animal-derived component.

13. The preparation method according to any one of claims 1 to 12, wherein the pluripotent stem cells are induced pluripotent stem cells.

14. The preparation method according to claim 13, wherein the induced pluripotent stem cells are human induced pluripotent stem cells.

15. A vascular endothelial progenitor cell obtained by the preparation method according to any one of claims 1 to 14.

16. The vascular endothelial progenitor cell according to claim 15, which has a purity of greater than or equal to 90%.

17. A cell preparation comprising the vascular endothelial progenitor cell according to claim 15 or 16.

18. Use of the vascular endothelial progenitor cell according to claim 15 or 16 for construction of a blood vessel in vitro or construction of a human blood-brain barrier model.

19. An extended culture method for vascular endothelial progenitor cells, comprising a step of culturing vascular endothelial progenitor cells in the presence of a ROCK inhibitor, a GSK-3β inhibitor, and a TGF-β receptor inhibitor, in addition to basic fibroblast growth factor and epidermal growth factor.

20. The extended culture method according to claim 19, wherein the ROCK inhibitor is Y-27632, the GSK-3β inhibitor is CHIR 99021, and the TGF-β receptor inhibitor is A 83-01.

21. The extended culture method according to claim 19 or 20, wherein the vascular endothelial progenitor cells are cells obtained by inducing differentiation of pluripotent stem cells.

22. The extended culture method according to claim 21, wherein the pluripotent stem cells are induced pluripotent stem cells.

23. The extended culture method according to claim 22, wherein the induced pluripotent stem cells are human induced pluripotent stem cells.

24. The extended culture method according to claim 19 or 20, wherein the vascular endothelial progenitor cells are cells prepared by the preparation method according to any one of claims 1 to 14.

25. The extended culture method according to claim 19 or 20, wherein the vascular endothelial progenitor cells are vascular endothelial progenitor cells collected in advance from a living body or cells obtained by maintaining or proliferating the vascular endothelial progenitor cells in vitro.

26. The extended culture method according to any one of claims 19 to 25, wherein the step is performed under conditions free of a xenogeneic animal-derived component.

# Fig. 1

# Fig. 2

**A**

TrypLE Select treatment
(45 to 60 seconds)

Tap several times strongly

Wash three times with PBS
( + suck cells around
culture dish )

TrypLE Select treatment
(6 to 12 minutes)

Obtain purified EPCs

Reseeding

**B**

**C**

**D**

Acetylated LDL uptake

96.2 ± 0.7 %

**E**

Tube formation assay

# Fig. 3

**A**

| | Additive component | Day 0 CHIR99021 | Day 1 bFGF | Day 2 VEGF & BMP4 | | Day 5 VEGF & bFGF | Day 8 |
|---|---|---|---|---|---|---|---|
| Normal conditions | Medium | Modified DMEM F12 | | | | Modified human endothelial SFM | |
| | Coat | Vitronectin-N | | | | Gelatin | |
| Xeno Free condition | Medium | Modified DMEM F12 | | | | Modified medium200 + Hydrocortisone | |
| | Coat | Vitronectin-N | | | | Vitronectin-N | |

**B**

Relative mRNA expression level

CD31    VE-Cad    vWF    CD34    FLK1

(Normal / XF)

**C**

CD31  97.4 ± 2.1%    VE-Cad  97.2 ± 1.4%    MERGE    CD34  92.5 ± 1.6%

**D**

Acetylated LDL uptake

96.8 ± 1.2 %

**E**

Tube formation assay

## Fig. 4

## Fig. 5

**Fig. 6**

**Fig. 7**

**Fig. 8**

**Fig. 9**

Fig. 10

Fig. 11

<div align="center">

**INTERNATIONAL SEARCH REPORT**

</div>

| | International application No. |
|---|---|
| | PCT/JP2020/005255 |

**A. CLASSIFICATION OF SUBJECT MATTER**
Int.Cl. A61P9/00(2006.01)n, C12N5/071(2010.01)i, C12N5/0735(2010.01)i, C12N5/10(2006.01)i, A61K35/44(2015.01)n
FI: C12N5/071, C12N5/0735, C12N5/10, A61K35/44, A61P9/00
According to International Patent Classification (IPC) or to both national classification and IPC

**B. FIELDS SEARCHED**

Minimum documentation searched (classification system followed by classification symbols)
Int.Cl. A61P9/00, A61K35/44, C12N5/071, C12N5/0735, C12N5/10

Documentation searched other than minimum documentation to the extent that such documents are included in the fields searched
Published examined utility model applications of Japan     1922-1996
Published unexamined utility model applications of Japan     1971-2020
Registered utility model specifications of Japan     1996-2020
Published registered utility model applications of Japan     1994-2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X | JP 2018-110548 A (NAGOYA CITY UNIV) 19.07.2018 (2018-07-19), claims, paragraphs [0005], [0015]-[0031], examples | 1, 5-7, 11-18 |
| X | SRIRAM, G. et al., Efficient differentiation of human embryonic stem cells to arterial and venous endothelial cells under feeder- and serum- free conditions, Stem Cell Research & Therapy, 2015, vol. 6, no. 261, pp. 1-17, supplementary methods, abstract, fig. 2, p. 3, left column, first paragraph to p. 5, left column, first paragraph, fig. 3, p. 6, right column, first paragraph to p. 7, left column, first paragraph, p. 9, left column, second paragraph, p. 15, left column, second paragraph, column of supplementary methods | 15-18 |

☒   Further documents are listed in the continuation of Box C.     ☒   See patent family annex.

| | |
|---|---|
| \*   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 13.04.2020 | 21.04.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/005255

C (Continuation).   DOCUMENTS CONSIDERED TO BE RELEVANT

| Category* | Citation of document, with indication, where appropriate, of the relevant passages | Relevant to claim No. |
|---|---|---|
| X | NGUYEN, M. T. X. et al., Differentiation of human embryonic stem cells to endothelial progenitor cells on laminins in defined and xeno-free systems, Stem Cell Reports, 2016, vol. 7, pp. 802-816, abstract, p. 803, left column, third paragraph to p. 806, left column, third paragraph, fig. 1, 3, p. 812, left column, first paragraph, right column, second paragraph | 1, 5-18 |
| A | JANG, S. et al., Induced pluripotent stem cell-derived endothelial cells: Overview, Current Advances, Applications, and Future Directions, The American Journal of Pathology, 2019.01.14, vol. 189, no. 3, pp. 502-512, abstract, p. 503, right column, third paragraph to p. 509, right column, third paragraph | 1-18 |
| A | 青木啓将等，異種由来成分不含条件下における iPS 細胞由来血管内皮前駆細胞の作出，第 17 回日本再生医療学会総会 (抄録)，2018.02.23, p.371, column of [0-22-3], columns of [purpose], [method], [results/discussion], (The 17th Congress of the Japanese Society for Regenerative Medicine(abstract)) non-official translation (AOKI, Keisho et al., Production of iPS cell-derived vascular endothelial progenitor cells under the xenogeneic component-free condition) | 14 |
| A | JP 2005-507258 A (CENTRE NATIONAL DE LA RECHERCHE SCIENTIFIQUE) 17.03.2005 (2005-03-17), claims, examples | 1-18 |
| A | JP 2013-521797 A (KYOTO UNIVERSITY) 13.06.2013 (2013-06-13), claims, examples | 1-18 |
| A | JP 2003-250820 A (MUROHARA, Toyoaki, ASAHI MEDICAL CO., LTD.) 09.09.2003 (2003-09-09), claim 4, paragraph [0024], etc. | 1-18 |
| T | AOKI, Hiromasa et al., Efficient differentiation and purification of human induced pluripotent stem cell-derived endothelial progenitor cells and expansion with the use of inhibitors of ROCK, TGF-β, and GSK3β, Heliyon, 2020.03.03, vol.6, e03493, whole document | 1-18 |
| A | JOO, H. J. et al., ROCK Suppression Promotes Differentiation and Expansion of Endothelial Cells from Embryonic Stem Cell-Derived Flk1(+) Mesodermal Precursor Cells, Blood, 2012, vol. 120, no. 13, pp. 2733-2744, particularly, abstract | 19-26 |

Form PCT/ISA/210 (second sheet) (January 2015)

| | International application No. |
|---|---|
| | PCT/JP2020/005255 |

C (Continuation).   DOCUMENTS CONSIDERED TO BE RELEVANT

| Category* | Citation of document, with indication, where appropriate, of the relevant passages | Relevant to claim No. |
|---|---|---|
| A | MASCKAUCHAN, T. N. H. et al., Wnt/b-catenin signaling induces proliferation, survival and Interleukin-8 in human endothelial cells, Angiogenesis, 2005, vol.8, pp. 43-51, particularly, abstract | 19-26 |
| A | JAMES, D. et al., Expansion and maintenance of human embryonic stem cell-derived endothelial cells by TGFbeta Inhibition Is Id1 Dependent, Nature Biotechnology, 2010, vol. 28, pp. 161-166, particularly, abstract | 19-26 |
| A | ZHANG, R. R. et al., Human iPSC-derived posterior gut progenitors are expandable and capable of forming gut and liver organoids, Stem Cell Reports, 2018, vol. 10, pp. 780-793, particularly, abstract | 19-26 |
| A | KATSUDA, T. et al., Conversion of terminally committed hepatocytes to culturable bipotent progenitor cells with regenerative capacity, cell stem cell, 2017, vol. 20, pp. 41-55, particularly, abstract | 19-26 |
| A | OKAE, H., et al., Derivation of Human trophoblast stem cells, Cell Stem Cell, 2018, vol. 22, pp. 50-63, particularly, abstract | 19-26 |

Form PCT/ISA/210 (second sheet) (January 2015)

## INTERNATIONAL SEARCH REPORT

| International application No. |
| --- |
| PCT/JP2020/005255 |

**Box No. II    Observations where certain claims were found unsearchable (Continuation of item 2 of first sheet)**

This international search report has not been established in respect of certain claims under Article 17 (2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
Document 1: JP 2018-110548 A (NAGOYA CITY UNIV) 19.07.2018 (2018-07-19), claims, paragraphs [0005], [0015]-[0031], examples
(Family: none)

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet) (January 2015)

**INTERNATIONAL SEARCH REPORT**

International application No.

PCT/JP2020/005255

**Box No. III**      **Observations where unity of invention is lacking (Continuation of item 3 of first sheet)**

This International Searching Authority found multiple inventions in this international application, as follows:

The claims are classified into two groups of inventions indicated below.

(Invention 1) Claims 1-18 Document 1 (particularly, see claims, paragraph [0022], etc.) discloses a method for preparing endothelial progenitor cells (EPCs) through induction of differentiation of induced pluripotent stem cells, wherein in the method, a cell population after induction of differentiation is inoculated on a culture surface, and suspension sperm cells that are non-adherent to the culture surface are removed, thereby selectively culturing the EPCs (corresponding to a step for purifying EPCs by means of the difference in adhesive capacity). As such, claim 1 lacks novelty in the light of document 1, and thus does not have a special technical feature.

However, claim 2 which is a dependent claim of claim 1 has a special technical feature of carrying out, as "a step for purifying endothelial progenitor cells by means of the difference in adhesive capacity between endothelial progenitor cells and other cells that form the cell population obtained in the step (1)", "(2-1) a step for detaching said other cells from the culture surface by a first detachment treatment using a first cell dissociation solution and removing said other cells" and "(2-2) a step for detaching said endothelial progenitor cells from the culture surface by a second detachment treatment using a second cell dissociation solution, the second detachment treatment having a higher cell detachment ability than the first cell detachment treatment, and recovering said endothelial progenitor cells". Claims 3-4 also have the same special technical feature as claim 2. Consequently, claims 1-4 are classified as invention 1.

Claims 5-18 are dependent claims of claim 1 and linked to the invention in claim 1, and thus are classified as invention 1.

(Invention 2) Claims 19-26 Claims 19-26 and the invention classified as invention 1 do not have the same or corresponding special technical features. Claims 19-26 are not dependent claims of the invention classified as invention 1. Claims 19-26 are not substantially identical to or similarly closely related to any of the claims classified as invention 1. Consequently, claims 19-26 cannot be classified as invention 1. Claims 19-26 have a special technical feature which resides in "a method for expansion culture of endothelial progenitor cells, comprising a step for culturing endothelial progenitor cells in the presence of, in addition to basic fibroblast growth factor and epidermal growth factor, a ROCK inhibitor, a GSK-3β inhibitor and a TGF-β receptor inhibitor", and thus is classified as invention 2.

Form PCT/ISA/210 (continuation of first sheet) (January 2015)

INTERNATIONAL SEARCH REPORT
Information on patent family members

International application No.

PCT/JP2020/005255

```
JP 2018-110548 A   19.07.2018    (Family: none)

JP 2005-507258 A   17.03.2005    US 2005/0106723 A1
                                 claims, examples
                                 WO 2003/038073 A2
                                 EP 1440147 A2

JP 2013-521797 A   13.06.2013    US 2013/0122586 A1
                                 claims, examples
                                 WO 2011/114719 A1
                                 EP 2547761 A1
                                 CN 102803475 A

JP 2003-250820 A   09.09.2003    US 2003/0180705 A1
                                 claim 5, paragraph [0096]
```

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019040117 A
- JP 2018110548 A
- WO 2008056779 A

### Non-patent literature cited in the description

- **MIEN T.X. et al.** Differentiation of Human Embryonic stem cells to endothelial progenitor cells on laminins in defined and xeno-free systems. *Stem cell reports,* 2016, vol. 7, 802-16
- **KYUNG-DAL CHOT. et al.** Hematopoietic and endothelial differentiation of human induced pluripotent stem cells. *Stem cells,* 2009, vol. 27, 559-67
- Manipulating the Mouse Embryo A Laboratory Manual. Cold Spring Harbor Laboratory Press, 1994
- **THOMSON, J. A. et al.** *Science,* 1998, vol. 282, 1145-1147
- **WILMUT et al.** *Nature,* 1997, vol. 385, 810
- **CIBELLI et al.** *Science,* 1998, vol. 280, 1256
- **AKIRA IRIYA et al.** *Protein Nucleic Acid and Enzyme,* 1999, vol. 44, 892
- **BAGUISI et al.** *Nature Biotechnology,* 1999, vol. 17, 456
- **WAKAYAMA et al.** *Nature,* 1998, vol. 394, 369
- *Nature Genetics,* 1999, vol. 22, 127
- *Proc. Natl. Acad. Sci. USA,* 1999, vol. 96, 14984
- **RIDEOUT III et al.** *Nature Genetics,* 2000, vol. 24, 109
- **TACHIBANA et al.** Human Embryonic Stem Cells Derived by Somatic Cell Nuclear Transfer. *Cell,* 2013
- **KIM et al.** *Science,* 2007, vol. 315, 482-486
- **NAKAJIMA et al.** *Stem Cells,* 2007, vol. 25, 983-985
- **KIM et al.** *Cell Stem Cell,* 2007, vol. 1, 346-352
- **REVAZOVA et al.** *Cloning Stem Cells,* 2007, vol. 9, 432-449
- **REVAZOVA et al.** *Cloning Stem Cells,* 2008, vol. 10, 11-24
- **STRELCHENKO N. et al.** *Reprod Biomed Online.,* 2004, vol. 9, 623-629
- **KLIMANSKAYA I. et al.** *Nature,* 2006, vol. 444, 481-485
- **CHUNG Y. et al.** *Cell Stem Cell,* 2008, vol. 2, 113-117
- **ZHANG X. et al.** *Stem Cells,* 2006, vol. 24, 2669-2676
- **WASSARMAN, P.M. et al.** *Methods in Enzymology,* 2003, vol. 365
- **MATSUI et al.** *Cell,* 1992, vol. 70, 841-847
- **SHAMBLOTT et al.** *Proc. Natl. Acad. Sci. USA,* 1998, vol. 95 (23), 13726-13731
- **TURNPENNY et al.** *Stem Cells,* 2003, vol. 21 (5), 598-609
- **TAKAHASHI K ; YAMANAKA S.** *Cell,* 2006, vol. 126 (4), 663-676
- **TAKAHASHI, K et al.** *Cell,* 2007, vol. 131 (5), 861-72
- **YU J et al.** *Science,* 2007, vol. 318 (5858), 1917-1920
- **NAKAGAWA M et al.** *Nat.Biotechnol.,* 2008, vol. 26 (1), 101-106
- **KIM J B et al.** *Nature,* 2008, vol. 454 (7204), 646-650
- **KIM J B et al.** *Cell,* 2009, vol. 136 (3), 411-419
- **ZHOU H ; WU S ; JOO JY et al.** *Cell Stem Cell,* 2009, vol. 4, 381-384
- **KIM D ; KIM CH ; MOON JI et al.** *Cell Stem Cell,* 2009, vol. 4, 472-476
- **HUANGFU D et al.** *Nat. Biotechnol.,* 2008, vol. 26 (7), 795-797
- **HUANGFU D et al.** *Nat. Biotechnol.,* 2008, vol. 26 (11), 1269-1275
- **SILVA J et al.** *PLoS. Biol.,* 2008, vol. 6 (10), e 253
- **STADTFELD M et al.** *Science,* 2008, vol. 322 (5903), 945-949
- **OKITA K et al.** *Science,* 2008, vol. 322 (5903), 949-953
- **WOLTJEN K ; MICHAEL IP ; MOHSENI P et al.** *Nature,* 2009, vol. 458, 766-770
- **KAJI K ; NORRBY K ; PAC A et al.** *Nature,* 2009, vol. 458, 771-775
- **YUSA K ; RAD R ; TAKEDA J et al.** *Nat Methods,* 2009, vol. 6, 363-369
- **YU J ; HU K ; SMUGA-OTTO K ; TIAN S et al.** *Science,* 2009, vol. 324, 797-801

- **SRIRAM G et al.** Efficient differentiation of human embryonic stem cells to arterial and venous endothelial cells under feeder- and serum-free conditions. *Stem Cell Research & Therapy,* 2015, vol. 6, 261
- **JAMES D. et al.** Expansion and maintenance of human embryonic stem cell-derived endothelial cells by TGFbeta inhibition is Id1 dependent. *Nature Biotechnology,* 2010, vol. 28 (2), 161-6
- **MIEN T.X. et al.** Differentiation of Human Embryonic stem cells to endothelial progenitor cells on laminins in defined and xeno-free systems. *Stem cell reports.,* 2016, vol. 7, 802-16

- **NAKAGAWA S ; MARIA A. D ; NAKAO S ; HONDA M ; HAYASHI M ; NAKAOKE R ; KATAOKA Y ; NIWA M.** *Cellular and Molecular Neurobiology.,* 2007, vol. 27, 687-694
- **IKUNO T. et al.** Efficient and robust differentiation of endothelial cells from human induced pluripotent stem cells via lineage control with VEGF and cyclic AMP. *PLOS ONE,* 2017, vol. 12, 3
- **LI S. et al.** Inhibition of cell growth and induction of inflammation by endosulfan in HUVEC-C cells. *Environmental Toxicology.,* 2016, vol. 12, 1785-1795
- **LIAN X. et al.** Efficient differentiation of human pluripotent stem cells to endothelial progenitors via small-molecule activation of WNT signaling. *Stem cell reports.,* 2014, vol. 3 (5), 804-16